(19) Europäisches Patentamt
European Patent Office
Office européen des brevets

(11) EP 1 562 913 B1

(12) **EUROPÄISCHE PATENTSCHRIFT**

(45) Veröffentlichungstag und Bekanntmachung des Hinweises auf die Patenterteilung:
**13.07.2011 Patentblatt 2011/28**

(21) Anmeldenummer: **03810409.7**

(22) Anmeldetag: **25.10.2003**

(51) Int Cl.:
***C07D 239/74*** *(2006.01)* ***A61K 31/517*** *(2006.01)*
***A61P 31/22*** *(2006.01)*

(86) Internationale Anmeldenummer:
**PCT/EP2003/011880**

(87) Internationale Veröffentlichungsnummer:
**WO 2004/041790 (21.05.2004 Gazette 2004/21)**

(54) **SUBSTITUIERTE CHINAZOLINE ALS ANTIVIRALE MITTEL, INSBESONDERE GEGEN CYTOMEGALOVIREN**

SUBSTITUTED QUINAZOLINES AS ANTIVIRAL AGENTS, ESPECIALLY AGAINST CYTOMEGALOVIRUSES

QUINAZOLINES SUBSTITUEES UTILISEES COMME AGENTS ANTIVIRAUX, EN PARTICULIER CONTRE DES CYTOMEGALOVIRUS

(84) Benannte Vertragsstaaten:
**DE ES FR GB IT**

(30) Priorität: **08.11.2002 DE 10251914**

(43) Veröffentlichungstag der Anmeldung:
**17.08.2005 Patentblatt 2005/33**

(73) Patentinhaber: **AiCuris GmbH & Co. KG**
**42117 Wuppertal (DE)**

(72) Erfinder:
- **WUNBERG, Tobias**
  **2371 Hinterbrühl (DE)**
- **BAUMEISTER, Judith**
  **2800 Mechelen (BE)**
- **JESKE, Mario**
  **42699 Solingen (DE)**
- **NIKOLIC, Susanne**
  **4051 Basel (CH)**
- **SÜSSMEIER, Frank**
  **42277 Wuppertal (DE)**
- **ZIMMERMANN, Holger**
  **42111 Wuppertal (DE)**
- **GROSSER, Rolf**
  **51373 Leverkusen (DE)**
- **HENNINGER, Kerstin**
  **42289 Wuppertal (DE)**
- **HEWLETT, GUY**
  **42781 Haan (DE)**
- **KELDENICH, Jörg**
  **42113 Wuppertal (DE)**
- **LANG, Dieter**
  **42553 Velbert (DE)**
- **TSE-I, Lin**
  **2800 Mechelen (BE)**

(74) Vertreter: **Witte, Weller & Partner**
**Postfach 10 54 62**
**70047 Stuttgart (DE)**

(56) Entgegenhaltungen:
**WO-A-99/41253**

- **WANG, FENGJLANG ET AL: "Solid-phase synthesis of 3,4-dihydroquinazoline" TETRAHEDRON LETTERS (1997), 38(50), 8651-8654 , XP004097142**
- **SAITO, TAKAO ET AL: "A facile and efficient carbodiimide-mediated synthesis of dihydroquinazolines via a tandem nucleophilic addition-intramolecular hetero conjugate addition annulation strategy" TETRAHEDRON LETTERS (1996), 37(2), 209-12 , XP004030439**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft substituierte Chinazoline und Verfahren zu ihrer Herstellung sowie ihre Verwendung zur Herstellung von Arzneimitteln zur Hebandlung und/oder Prophylaxe von Krankheiten, insbesondere zur Verwendung als antivirale Mittel, insbesondere gegen Cytomegaloviren.

Die Synthese von Chinazolinen ist beschrieben in Saito T., et al. Tetrahedron Lett., 1996, 37, 209-212.

Auf dem Markt sind zwar strukturell andersartige, antiviral wirkende Mittel vorhanden, es kann aber regehnäßig zu einer Resistenzeatwicklung kommen. Neue Mittel für eine bessere und wirksame Therapic sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Chinazoline antiviral hochwirksam sind.

Gegenstand der Erfindung sind Verbindungen der Formel

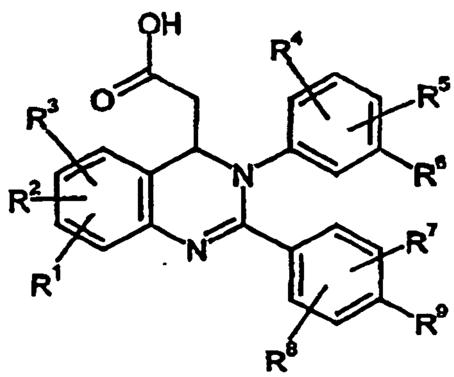

(I),

in welcher

$R^1$, $R^2$ und $R^3$ unabhängig voneinander für Wasserstoff, Alkyl, Alkoxy, Carboxyl, Alkylcarbonyl, Alkoxycarbonyl, Aminocarbonyl, Trifluormethyl, Halogen, Cyano, Hydroxy oder Nitro stehen,

$R^4$ und $R^5$ unabhängig voneinander für Wasserstoff, Alkyl, Alkoxy, Cyano, Halogen, Nitro, Trifluormethyl oder Trifluormethoxy stehen,

$R^6$ für Alkyl, Cyano, Halogen, Nitro oder Trifluormethyl steht,

$R^7$ und $R^8$ unabhängig voneinander für Wasserstoff, Halogen, Alkyl oder Alkoxy stehen und

$R^9$ für Aryl oder 1,3-Benzodioxol-5-yl steht, worin Aryl und 1,3-Benzodioxol-5-yl substituiert sein können mit 1 bis 3 Substituenten, wobei die Substituenten unabhängig voneinander ausgewählt werden aus der Gruppe, bestehend aus Alkoxy, Alkylthio, Carboxyl, Alkylcarbonyl, Alkoxycarbonyl, Aminocarbonyl, Trifluormethyl, Halogen, Carbamoyl, Cyano, Hydroxy, Amino, Alkylamino, Nitro und gegebenenfalls Hydroxy-substituiertes Alkyl,

und ihre Salze, ihre Solvate und die Solvate ihrer Salze.

Erfindungsgemäße Verbindungen sind die Verbindungen der Formel (I) und deren Salze, Solvate und Solvate der Salze, nachfolgend als Ausfiihrungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylcarbonyl und Alkoxycarbonyl stehen für einen linearen oder verzweigten Alkylrest mit in der Regel 1 bis 6 ("$C_1$-$C_6$-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylcarbonyl steht beispielhaft und vorzugsweise für Acetyl und Propanoyl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N*,*N*-Dimethylamino, *N*,*N*-Dietilylamino, *N*-Ethyl-*N*-methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino. $C_1$-$C_3$-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Halogen steht für Fluor, Chlor, Brom und Jod.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung eine Enantiomerenüberschuss (enantiomeric excess) von mehr als 90 % verstanden wird (> 90 %ee).

Bevorzugt im Rahmen der vorliegenden Erfindungen sind Verbindungen der Formel (I),
in welcher
$R^1$, $R^2$ und $R^3$ unabhängig voneinander für Wasserstoff, Alkyl, Alkoxy, Carboxyl, Alkylcarbonyl, Alkoxycarbonyl, Trifluormethyl, Halogen, Cyano, Hydroxy oder Nitro stehen,
$R^4$ und $R^5$ unabhängig voneinander für Wasserstoff, Alkyl, Alkoxy, Cyano, Halogen, Nitro oder Trifluormethyl stehen,
$R^6$ für Alkyl, Cyano, Halogen, Nitro oder Trifluormethyl steht,
$R^7$ und $R^8$ unabhängig voneinander für Wasserstoff, Halogen, Alkyl oder Alkoxy stehen und
$R^9$ für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Carboxyl, Alkylcarbonyl, Alkoxycarbonyl, Trifluormethyl, Halogen, Carbamoyl, Cyano, Hydroxy, Amino, Alkylamino und Nitro,
und ihre Salze, ihre Solvate und die Solvate ihrer Salze.

Bevorzugt im Rahmen der vorliegenden Erfindungen sind Verbindungen der Formel (I),
in welcher
$R^1$, $R^2$ und $R^3$ unabhängig voneinander für Wasserstoff, Fluor, Chlor, Cyano, Hydroxy, Aminocarbonyl oder Nitro stehen,
$R^4$ und $R^5$ unabhängig voneinander für Wasserstoff, Fluor, Alkyl oder Alkoxy stehen,
$R^6$ für Trifluormethyl, iso-Propyl oder tert.-Butyl steht,
$R^7$ und $R^8$ unabhängig voneinander für Wasserstoff, Halogen, $C_1$-$C_3$-Alkyl oder $C_1$-$C_3$-Alkoxy stehen und
$R^9$ für Phenyl oder 1,3-Benzodioxol-5-yl steht, worin Phenyl substituiert sein kann mit 1 bis 3 Substituenten, wobei die Substituenten unabhängig voneinander ausgewählt werden aus der Gruppe, bestehend aus $C_1$-$C_6$-Alkyl, $C_1$-$C_6$-Alkoxy, Carboxyl, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino und Nitro,
und ihre Salze, ihre Solvate und die Solvate ihrer Salze.

Bevorzugt im Rahmen der vorliegenden Erfindungen sind Verbindungen der Formel (I),
in welcher

$R^1$, $R^2$ und $R^3$ unabhängig voneinander für Wasserstoff, Fluor, Chlor, Cyano, Hydroxy, Aminocarbonyl oder Nitro stehen,
$R^4$ und $R^5$ unabhängig voneinander für Wasserstoff, Fluor, Alkyl oder Alkoxy stehen,
$R^6$ für Trifluormethyl, iso-Propyl oder tert.-Butyl steht,
$R^7$ und $R^8$ unabhängig voneinander für Wasserstoff, Halogen, $C_1$-$C_3$-Alkyl oder $C_1$-$C_3$-Alkoxy stehen und
$R^9$ für Phenyl steht, worin Phenyl substituiert sein kann mit 1 bis 3 Substituenten, wobei die Substituenten unabhängig voneinander ausgewählt werden aus der Gruppe, bestehend aus $C_1$-$C_6$-Alkyl, $C_1$-$C_6$-Alkoxy, Carboxyl, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino und Nitro,
und ihre Salze, ihre Solvate und die Solvate ihrer Salze.

Bevorzugt im Rahmen der vorliegenden Erfindungen sind auch Verbindungen der Formel (I),
in welcher
$R^1$ und $R^2$ für Wasserstoff stehen,
$R^3$ für Fluor steht,
$R^4$ und $R^5$ unabhängig voneinander für Wasserstoff, Fluor oder Alkoxy stehen,
$R^6$ für Trifluormethyl steht,
$R^7$ und $R^8$ für Wasserstoff stehen und
$R^9$ für Phenyl steht, worin Phenyl substituiert sein kann mit 1 oder 2 Substituenten, wobei die Substituenten unabhängig voneinander ausgewählt werden aus der Gruppe, bestehend aus Methyl, Methoxy, Fluor und Chlor,
und ihre Salze, ihre Solvate und die Solvate ihrer Salze.

Bevorzugt im Rahmen der vorliegenden Erfindungen sind auch Verbindungen der Formel (I), in welcher $R^1$ und $R^2$ für Wasserstoff stehen.

Bevorzugt im Rahmen der vorliegenden Erfindung sind auch Verbindungen der Formel (I), in welcher $R^3$ an das Kohlenstoffatom in Position 6 oder in Position 8 des Chinazolin-Gerüsts gebunden ist.

Bevorzugt im Rahmen der vorliegenden Erfindung sind auch Verbindungen der Formel (I), in welcher $R^3$ an das Kohlenstoffatom in Position 8 des Chinazolin-Gerüsts gebunden ist.

Bevorzugt im Rahmen der vorliegenden Erfindungen sind auch Verbindungen der Formel (I), in welcher $R^3$ für Fluor steht, insbesondere für ein an das Kohlenstoffatom in Position 8 des Chinazolin-Gerüstes gebundenes Fluor.

Bevorzugt im Rahmen der vorliegenden Erfindungen sind auch Verbindungen der Formel (I), in welcher $R^4$ und $R^5$ für Wasserstoff stehen.

Bevorzugt im Rahmen der vorliegenden Erfindungen sind auch Verbindungen der Formel (I), in welcher $R^4$ für Wasserstoff und $R^5$ für Fluor oder Alkoxy stehen. Bevorzugt im Rahmen der vorliegenden Erfindungen sind auch Verbindungen der Formel (I), in welcher $R^6$ für Trifluormethyl steht.

Bevorzugt im Rahmen der vorliegenden Erfindungen sind auch Verbindungen der Formel (I), in welcher $R^6$ für iso-Propyl oder tert.-Butyl steht.

Bevorzugt im Rahmen der vorliegenden Erfindungen sind auch Verbindungen der Formel (I), in welcher $R^7$ und $R^8$ für Wasserstoff stehen.

Bevorzugt im Rahmen der vorliegenden Erfindungen sind auch Verbindungen der Formel (I), in welcher $R^9$ für Phenyl steht, worin Phenyl substituiert sein kann mit 1 bis 2 Substituenten, wobei die Substituenten unabhängig voneinander ausgewählt werden aus der Gruppe bestehend aus Methyl, Methoxy, Fluor und Chlor.

Gegenstand der Erfindung ist weiterhin ein Verfahren zur Herstellung der Verbindungen der Formel (I), wobei Verbindungen der Formel

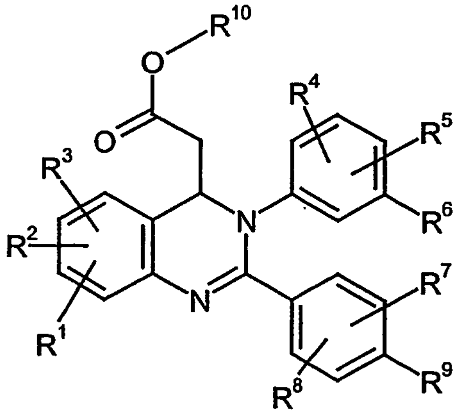

(II),

in welcher
$R^1$, $R^2$, $R^3$, $R^4$, $R^5$, $R^6$, $R^7$, $R^8$ und $R^9$ die oben angegebene Bedeutung haben, und
$R^{10}$ für Alkyl, bevorzugt Methyl oder Ethyl, steht,
mit Basen umgesetzt werden.

Die Umsetzung erfolgt im allgemeinen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Halogenkohlenwasserstoffe wie Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Gemischen von Lösungsmitteln, bevorzugt ist Dioxan oder Tetrahydrofuran.

Die Verbindungen der Formel (II) sind bekannt oder können hergestellt werden, indem Verbindungen der Formel

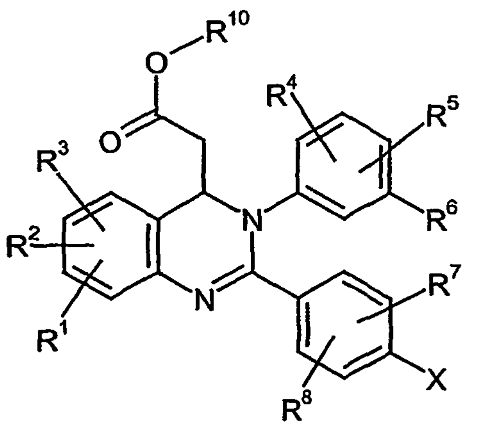

(III),

in welcher
$R^1$, $R^2$, $R^3$, $R^4$, $R^5$, $R^6$, $R^7$, $R^8$ und $R^{10}$ die oben angegebene Bedeutung haben und
X für Halogen, bevorzugt Brom oder Chlor, steht,
mit Verbindungen der Formel

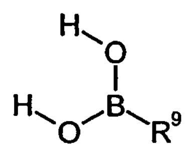

(IV),

in welcher
$R^9$ die oben angegebene Bedeutung hat,
unter Suzuki-Kupplungsbedingungen umgesetzt werden.

Die Umsetzung erfolgt im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis 130˚C bei Normaldruck.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)-acetat,Palladium(II)acetat/TriscyclohexylphosphinoderBis-(diphenylphosphanferrocenyl)-palladium-(II)-chlorid.

Zusatzreagenzien sind beispielsweise Kaliumacetat, Cäsium-, Kalium- oder Natriumcarbonat, Kalium-tert.-butylat, Cäsiumfluorid oder Kaliumphosphat durchgeführt, bevorzugt sind Zusatzreagenzien wie z.B. Kaliumacetat und/oder wässrige Natriumcarbonatlösung.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Carbonsäureamide wie Dimethylformamid oder Dimethylacetamid, Alkylsulfoxide wie Dimethylsulfoxid, oder N-Methylpyrrolidon, bevorzugt ist Dioxan.

Die Verbindungen der Formel (IV) sind bekannt oder lassen sich nach bekannten Verfahren aus den entsprechenden Edukten synthetisieren.

Die Verbindungen der Formel (III) sind bekannt oder können hergestellt werden, indem Verbindungen der Formel

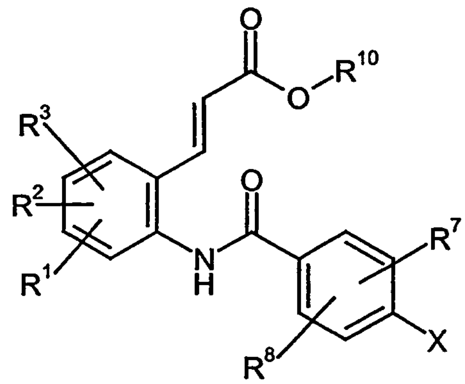

(V),

in welcher
$R^1$, $R^2$, $R^3$, $R^7$, $R^8$, $R^{10}$ und X die oben angegebene Bedeutung haben,
mit Verbindungen der Formel

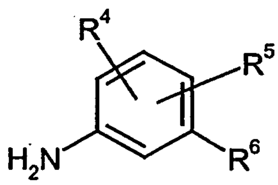

(VI),

in welcher
$R^4$, $R^5$ und $R^6$ die oben angegebene Bedeutung haben,
in Gegenwart von Phosphoroxychlorid umgesetzt werden.

Die Umsetzung erfolgt im Allgemeinen in inerten Lösungsmitteln, bevorzugt in einem Temperaturbereich von 50°C bis zum Rückfluss der Lösungsmittel bei Normaldruck.

Inerte Lösungsmittel sind beispielsweise Kohlenwasserstoffe wie Benzol, Xylol, Toluol, Hexan, Cyclohexan oder Erdölfraktionen, bevorzugt ist Toluol.

Alternativ können die Verbindungen der Formel (III) in einem zweistufigen Syntheseverfahren hergestellt werden. In der ersten Stufe werden die Verbindungen der Formel (V) mit Phosphoroxychlorid in einem inerten Lösungsmittel, bevorzugt ist Toluol, unter Rückfluss bei Normaldruck erhitzt. Das Lösungsmittel wird entfernt. In der zweiten Stufe werden die so erhaltenen Verbindungen mit Verbindungen der Formel (VI) in einem inerten Lösungsmittel, bevorzugt ist Toluol, ebenfalls unter Rückfluss bei Normaldruck umgesetzt.

Die Verbindungen der Formel (VI) sind bekannt oder lassen sich nach bekannten Verfahren aus den entsprechenden Edukten synthetisieren.

Die Verbindungen der Formel (V) sind bekannt oder können hergestellt werden, indem Verbindungen der Formel

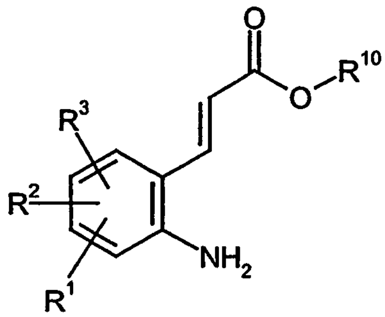

(VII),

in welcher
$R^1$, $R^2$, $R^3$ und $R^{10}$ die oben angegebene Bedeutung haben,
mit Verbindungen der Formel

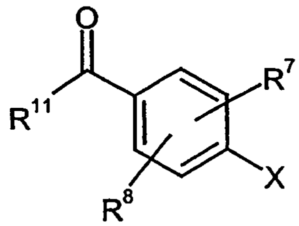

(VIII),

in welcher
$R^7$, $R^8$ und X die oben angegebene Bedeutung haben und
$R^{11}$ für Halogen, bevorzugt Chlor, Brom oder Iod, oder Hydroxy steht,
umgesetzt werden.

Im Falle, dass $R^{11}$ für Hydroxy steht,
erfolgt die Umsetzung im allgemeinen in inerten Lösungsmitteln, in Gegenwart von üblichen Kondensationsmitteln, gegebenenfalls in Gegenwart einer Base, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, 1,2-Dimethoxyethan, Glykoldimethylether oder Diethylenglykoldimethylether, Kohlenwasserstoffe wie Benzol, Xylol, Toluol, Hexan, Cyclohexan oder Erdölfraktionen, oder Carbonsäureamide wie Dünethylformamid oder Dimethylacetamid, Alkylnitrile wie Acetonitril, oder Heteroaromaten wie Pyridin, oder Ethylacetat, bevorzugt sind Tetrahydrofuran, 1,2-Dichlorethan oder Methylenchlorid.

Übliche Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethyl-aminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluoro-phosphat, oder O-(Benzotriazol-1-yl)-N,N,N',N'-tetra-methlyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N-tetramethyl-uroniumhexa-fluorophosphat (HATU), oder 1-Hydroxybenztriazol (HOBt), oder Benzotriazol-1-yloxylris(dimethylamino)-phosphoniumhexafluoro-phosphat (BOP), oder Mischungen aus diesen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Besonders bevorzugt ist die Kombination von N-(3-Dimethylaminoisopropyl)-N'-ethylcarbodiimid-Hydrochlorid (EDC), 1-Hydroxybenztriazol (HOBt) und Tri-ethylamin in Dimethylformamid oder Carbonyldiimidazol in 1,2-Dichlorethan.

Im Falle, dass $R^{11}$ für Halogen steht,

erfolgt die Umsetzung im allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, 1,2-Dimethoxyethan, Glykoldimethylether oder Diethylenglykoldimethylether, Kohlenwasserstoffe wie Benzol, Xylol, Toluol, Hexan, Cyclohexan oder Erdölfraktionen, oder Carbonsäureamide wie Dimethylformamid oder Dimethylacetamid, Alkylnitrile wie Acetonitril, oder Heteroaromaten wie Pyridin, oder Ethylacetat, bevorzugt sind Tetrahydrofuran, Dioxan oder Methylenchlorid.

Basen sind beispielsweise Alkalicarbonate wie Cäsiumcarbonat, Natrium- oder Kaliumcarbonat, oder andere Basen wie Triethylamin oder Diisopropylethylamin, bevorzugt Diisopropylethylamin oder Triethylamin.

Die Verbindungen der Formel (VIII) sind bekannt oder lassen sich nach bekannten Verfahren aus den entsprechenden Edukten synthetisieren.

Die Verbindungen der Formel (VII) sind bekannt oder lassen sich nach bekannten Verfahren aus den entsprechenden Edukten synthetisieren, beispielsweise durch eine Heck-Reaktion oder eine Wittig-Horner-Reaktion nach folgenden Syntheseschemata:

<u>Heck-Reaktion:</u>

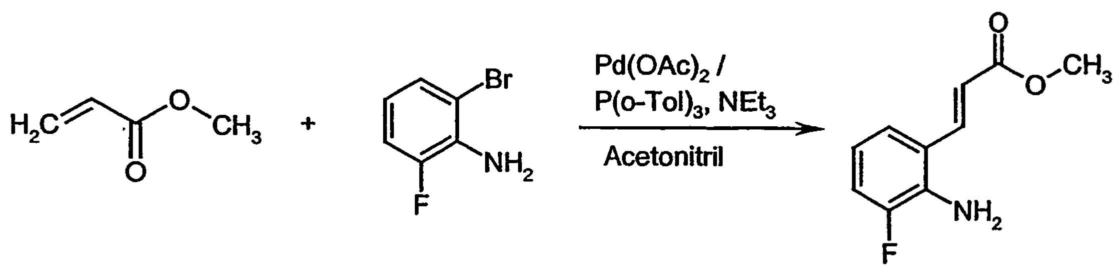


<u>Wittig-Horner-Reaktion:</u>

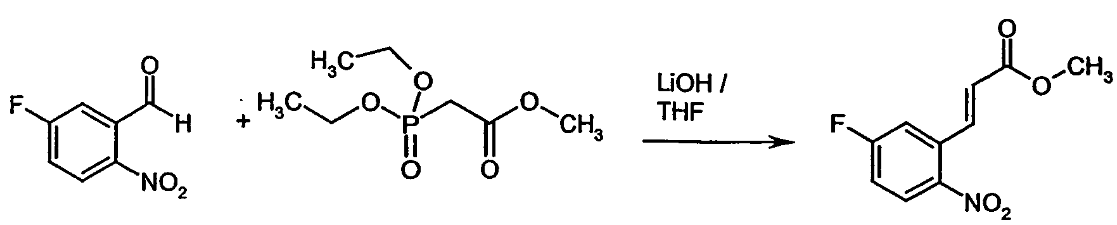


Die dafür benötigten Edukte sind bekannt oder lassen sich nach bekannten Verfahren aus den entsprechenden Edukten synthetisieren.

Die Herstellung der erfindungsgemäßen Verbindungen kann durch das folgende Syntheseschema verdeutlicht werden.

Syntheseschema:

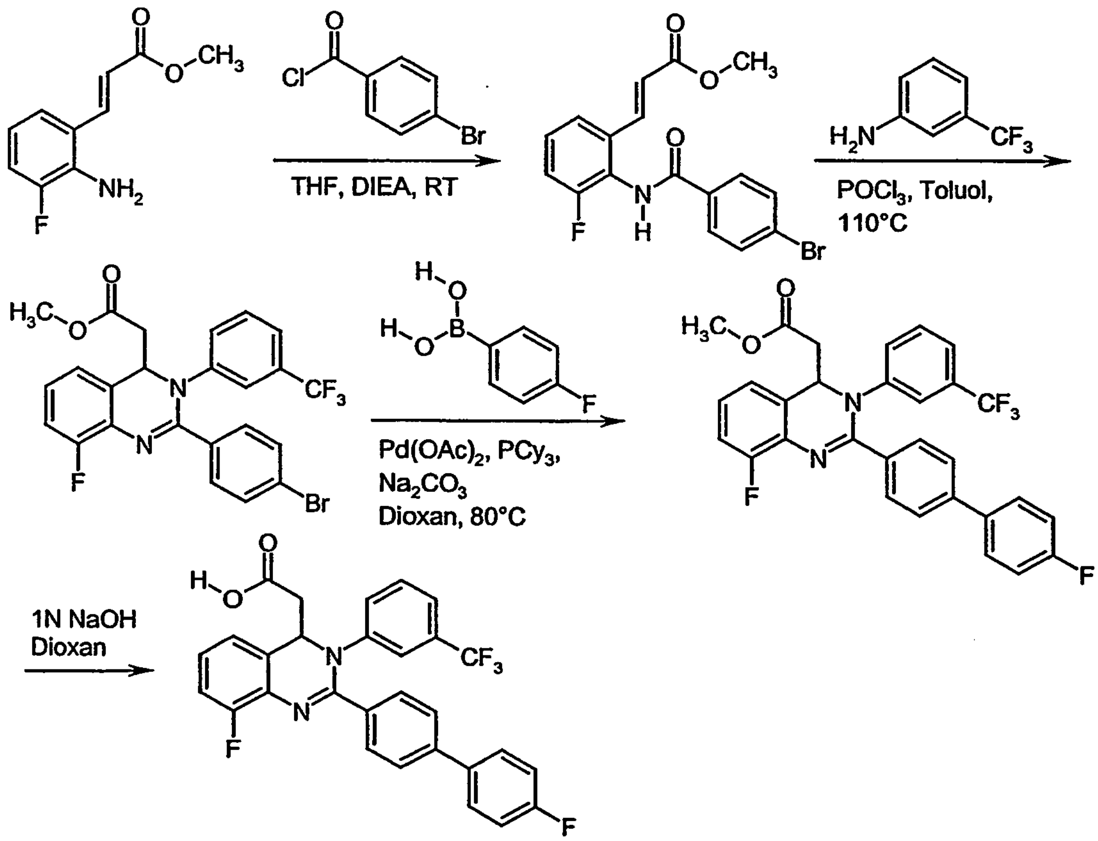


Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV). Sie sind somit geeignet zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Die Verbindungen der allgemeinen Formel (I) können aufgrund ihrer besonderen Eigenschaften zur Herstellung von Arzneimitteln, die zur Prophylaxe und/oder Behandlung von Krankheiten, insbesondere Virusinfektionen, geeignet sind, verwendet werden.

Als Indikationsgebiete können beispielsweise genannt werden:

1) Behandlung und Prophylaxe von HCMV-Infektionen bei AIDS-Patienten (Retinitis, Pneumonitis, gastrointestinale Infektionen).

2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

3) Behandlung und Prophylaxe von HCMV-Infektionen bei Neugeborenen und Kleinkindern.

4) Behandlung einer akuten HCMV-Infektion bei Schwangeren.

5) Behandlung der HCMV-Infektion bei immunsupprimierten Patienten bei Krebs und Krebs-Therapie.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Die erfindungsgemäßen Verbindungen können aufgrund ihrer pharmakologischen Eigenschaften allein und bei Bedarf auch in Kombination mit anderen Wirkstoffen, insbesondere antiviralen Wirkstoffen wie beispielsweise Gancyclovir oder Acyclovir, zur Behandlung und/oder Prävention von Virusinfektionen, insbesondere von HCMV-Infektionen, eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart-oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Im Allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## A. Beispiele

**Verwendete Abkürzungen:**

| | |
|---|---|
| $CD_3CN$ | Deuteroacetonitril |
| DC | Dünnschichtchromatographie |

(fortgesetzt)

| | |
|---|---|
| DCI | direkte chemische Ionisation (bei MS) |
| DCM | Dichlormethan |
| DIEA | *N*,*N*-Diisopropylethylamin (Hünig Base) |
| DMSO | Dimethylsulfoxid |
| DMF | *N,N*-Dimethylformamid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| h | Stunde |
| HPLC | Hochdruck-, Hochleistungsflüssigchromatographie |
| konz. | konzentriert |
| LC-MS | Flüssigchromatographie-gekoppelte Massenspektroskopie |
| LDA | Lithium-Diisopropylamid |
| MS | Massenspektroskopie |
| NMR | Kernresonanzspektroskopie |
| proz. | prozentig |
| RP-HPLC | Reverse Phase HPLC |
| RT | Raumtemperatur |
| $R_t$ | Retentionszeit (bei HPLC) |
| THF | Tetrahydrofuran |

**Allgemeine Methoden LC-MS und HPLC:**

**Methode 1 (HPLC):** Instrument: HP 1100 mit DAD-Detektion; Säule: Kromasil RP-18, 60 mm x 2 mm, 3.5 µm; Eluent A: 5 ml $HClO_4$/l Wasser, Eluent B: Acetonitril; Gradient: 0 min 2 % B, 0.5 min 2 % B, 4.5 min 90 % B, 6.5 min 90 % B; Fluss: 0.75 ml/min; Temp.: 30°C; UV-Detektion: 210 nm.

**Methode 2 (HPLC, Enantiomerentrennung):** Chiraler Kieselgelselektor KBD 6136 (10 µm, 350 x 30 mm) basierend auf dem Selektor Poly(N-methacryloyl-L-leucin-1-menthylamid); Temperatur: 24°C; Fluss. 50 ml/min; UV-Detektion: 254 nm; Probenaufgabe in Ethylacetat; Elutionsgemische aus iso-Hexan (A)/Ethylacetat (B), z. B.: Gradient: → 0 min 40 % B → 9.0 min 40 % B → 9.01 min 100 % B → 12.0 min 100 % B → 12.01 min 40 % B → 15 min 40 % B.

**Methode 3 (LCMS):** Instrument: Micromass Platform LCZ mit HPLC Agilent Serie 1100; Säule: Grom-SIL120 ODS-4 HE, 50 mm x 2.0 mm, 3 µm; Eluent A: 11 Wasser + 1 ml 50 %ige Ameisensäure, Eluent B: 11 Acetonitril + 1 ml 50 %ige Ameisensäure; Gradient: 0.0 min 100 % A → 0.2 min 100 % A → 2.9 min 30 % A → 3.1 min 10 %A → 4.5 min 10 % A; Ofen: 55°C; Fluss: 0.8 ml/min; UV-Detektion: 208-400 nm.

**Methode 4 (HPLC, präparative Trennung):** Säule: CromSil C18, 250x30; Fluss: 50 ml/min; Laufzeit: 38 min; Detektion: 210 nm; Eluent A = Wasser, Eluent B = Acetonitril; Gradient: 10 %B (3 min) -> 90 %B (31 min) -> 90% B (34 min) -> 10 % B (34.01 min).

**Methode 5 (HPLC):** Instrument: HP 1100 mit DAD-Detektion; Säule: Kromasil RP-18, 60 mm x 2 mm, 1.0 µl; Eluent A: 5 ml $HClO_4$/l Wasser, Eluent B: Acetonitril; Gradient: 0 min 2 % B, 0.5 min 2 % B, 4.5 min 90 % B, 9.0 min 90 % B; Fluss: 0.75 ml/min; Temp.: 30°C; UV-Detektion: 210 nm.

**Methode 6 (HPLC, Enantiomerentrennung):** Chiraler Kieselgelselektor ZWE 840B (10µm; Säule 250 * 20mm) basierend auf dem Selektor Poly(N-methacryloyl-L-leucin-(+)-3-aminomethylpinanylamid); Temperatur: 24°C; Fluss 25 ml/min; UV-Detektion: 280 nm; Probenaufgabe in iso-Hexan/Ethylacetat; Elutionsmittelgemisch aus iso-Hexan/Ethylacetat 7:3 (vol/vol).

**Methode 7 (LCMS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent B: Acetonitril + 0.05% Ameisensäure, Eluent A: Wasser + 0.05% Ameisensäure; Gradient: 0.0 min 5%B → 2.0 min 40%B → 4.5 min 90%B → 5.5 min 90%B; Ofen: 45°C; Fluss: 0.0 min 0.75 ml/min → 4.5 min 0.75 ml/min → 5.5 min 1.25 ml/min; UV-Detektion: 210 nm.

**Methode 8: (LCMS):** Instrument: Micromass Quattro LCZ, mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

**Methode 9 (HPLC, Enantiomerentrennung):** Chiraler Kieselgelselektor KBD 8361 (10µm; Säule 250 * 20mm) basierend auf dem Selektor Poly(N-methacryloyl-L-leucin-1-menthylamid); Temperatur: 24°C; Fluss 25 ml/min; UV-Detektion: 280 nm; Probenaufgabe in iso-Hexan/Ethylacetat; Elutionsmittelgemisch aus iso-Hexan/Ethylacetat 1:1 (vol/vol).

**Methode 10 (HPLC, Enantiomerentrennung):** Chiraler Kieselgelselektor KBD 6784 (10µm; Säule 250 * 20mm) basierend auf dem Selektor Poly(N-methacryloyl-L-leucin-2,4-dimethypentylamid); Temperatur: 24°C; Fluss 20 ml/min; UV-Detektion: 270 nm; Probenaufgabe in Methyl-tert.butylether; Elutionsmittel: Methyl-tert.-butylether.

**Ausgangsverbindungen**

**Allgemeine Arbeitsvorschrift [A]: Synthese von substituierten 2-Aminozimtsäurederivaten mittels Heck-Kupplung aus 2-halogensubstituierten Anilinen**

In einem Einhalskolben werden 1.0 Äquivalente eines Arylhalogenids mit 1.6 Acrylsäuremethylester, 2.0 Äquivalenten Triethylamin, 0.03 Äquivalenten Palladium(II)acetat und 0.03 Äquivalenten Tri-o-tolylphosphin in Acetonitril vorgelegt (ca. 1M-Lösung). Man lässt das Gemisch unter Rückfluss für 48 Stunden rühren. Nach beendeter Reaktion (Reaktionskontrolle mittels DC) wird das Lösemittel entfernt. Der Rückstand wird über Kieselgel mit Cyclohexan/Ethylacetat = 8:2 v/v chromatographisch gereinigt.

**Beispiel 1A**

(2E)-3-[2-Amino-3-fluorphenyl]-propensäuremethylester

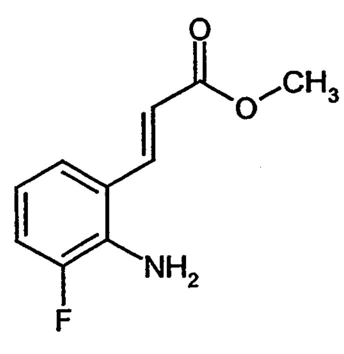

Ausgehend von 42.00 g (221.04 mmol) 2-Brom-6-fluoranilin werden nach der allgemeinen Arbeitsvorschrift [A] 29.66 g (68% d. Th.) Produkt erhalten.
HPLC (Methode 1): $R_t$= 4.14 min
MS (ESI-pos): m/z =196 $(M+H)^+$

**Beispiel 2A**

(2E)-3-[2-Amino-3-fluor-5-methylphenyl]-propensäuremethylester

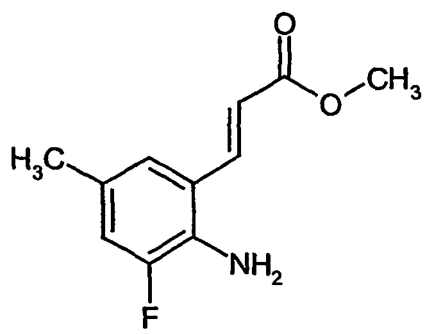

Ausgehend von 1.9 g (9.31 mmol) 2-Brom-4-methyl-6-fluoranilin werden nach der allgemeinen Arbeitsvorschrift [A] 502 mg (25% d. Th.) Produkt erhalten.
HPLC (Methode 1): $R_t$= 4.31 min
MS (ESI-pos): m/z = 210 $(M+H)^+$

**Allgemeine Arbeitsvorschrift [B]: Acylierung der 2-Aminozimtsäureester mit Benzoylchloriden**

In 200 ml THF werden 25.6 mmol des 2-Aminozimtsäuresters sowie 25.6 mmol Hünig Base vorgelegt, bei Raumtemperatur wird das Säurechlorid zugegeben und die Mischung 16 h gerührt. Anschließend wird das Lösungsmittel im Vakuum entfernt und der Rückstand mit Dichlormethan aufgenommen. Dabei bildet sich ein Niederschlag, der mit Dichlormethan verrührt und abgesaugt wird. Danach werden die Kristalle mit Wasser aufgeschlämmt, verrührt, abgesaugt und im Vakuum getrocknet.

**Beispiel 3A**

(2E)-3- {2-[(4-Brombenzoyl)amino]-3-fluorphenyl}-2-propensäuremethylester

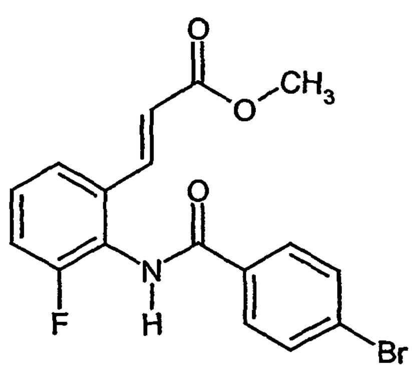

Ausgehend von 5.0 g (25.6 mmol) Aminozimtsäureester aus Beispiel 1A werden nach der allgemeinen Arbeitsvorschrift [B] 7.77 g (79% d. Th.) Produkt erhalten. HPLC (Methode 1): $R_t$= 4.48 min

**Beispiel 4A**

(2E)-3-{2-[(4-Brom-2-fluorbenzoyl)amino]-3-fluorphenyl}-2-propensäure-methylester

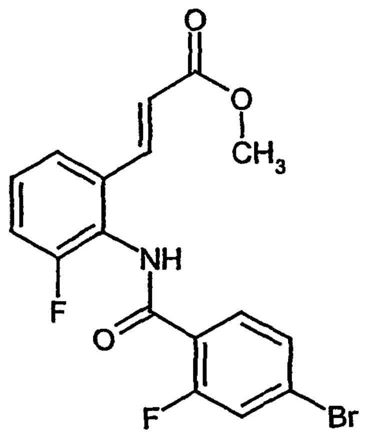

Ausgehend von 162 mg (0.83 mmol) Aminozimtsäureester aus Beispiel 1A werden nach der allgemeinen Arbeitsvorschrift [B] 148 mg (45% d. Th.) Produkt erhalten.
HPLC (Methode 1): $R_t$= 4.64 min

**Beispiel 5A**

(2E)-3-{2-[(4-Brombenzoyl)amino]-3-fluor-5-methylphenyl}-2-propensäure-methylester

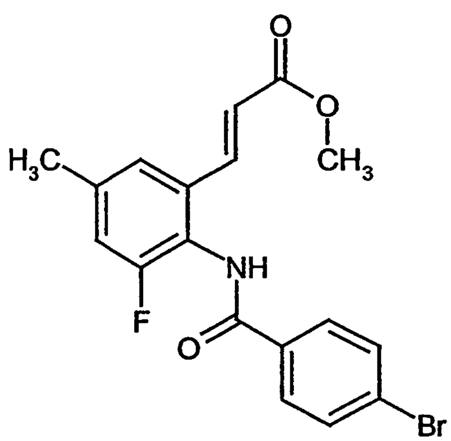

Ausgehend von 536 mg (2.56 mmol) Aminozimtsäureester aus Beispiel 2A werden nach der allgemeinen Arbeitsvorschrift [B] 700 mg (62% d. Th.) Produkt erhalten.
HPLC (Methode 1): $R_t$= 4.70 min
MS (DCI): m/z = 409 $(M+NH_4)^+$

**Allgemeine Arbeitsvorschrift [C]: Cylisierung der 2-Aminoacylzimtsäureester mit Anilinen**

Variante 1:

In 300 ml Toluol werden bei Raumtemperatur 79.3 mmol des 2-Aminoacylzimtsäuresters sowie 475.9 mmol des Anilins sowie 238.0 mmol Phosphoroxychlorid vorgelegt. Die Suspension wird unter intensivem Rühren 24-72 h unter Rückfluss erhitzt (Badtemperatur 120-125°C). Der Umsatz wird mittels Dünnschichtchromatogramm oder HPLC verfolgt, alle 24 h werden neue Mengen an Anilin und Phosphoroxychlorid hinzugegeben. Anschließend wird das Lösungsmittel im Vakuum entfernt und der Rückstand mit Dichlormethan aufgenommen. Das Produkt wird durch Chromatographie an Kieselgel mit Cyclohexan/Ethylacetat-Gemischen gereinigt. Sollte das Produkt während der Reinigung auf dem Kieselgel auskristallisieren, wird es gegebenenfalls nach Waschen des Kieselgels mit reinem Ethylacetat mit reinem Methanol isoliert.

Variante 2:

Alternativ zur Variante 1 wird zunächst der 2-Aminoacylzimtsäureester 24h mit Phosphoroxychlorid in Toluol unter Rückfluss umgesetzt, der Ansatz eingeengt, dann das Anilin zugegeben und die Reaktionsmischung erneut in Toluol 24h unter Rückfluss erhitzt. Die Aufarbeitung erfolgt wie unter Variante 1 beschrieben.

Variante 3:

Alternativ zu den Varianten 1 und 2 werden 1 Äquivatent 2-Acylaminozimtsäureester aus Beispiel 3A in Toluol suspendiert und mit 10 Äquivalenten Phosphorylchlorid versetzt. Nach Rühren über Nacht unter Rückfluss wird bis zur Trockene eingeengt und der Rückstand in Toluol aufgenommen. Anschließend wird mit 3 Äquivalenten Anilin in der Siedehitze versetzt und erneut unter Rückfluss über Nacht gerührt. Nach Abdestillieren des Lösungsmittels wird in Dichlormethan gelöst und mit 1N Salzsäure und Natriumhydrogencarbonat-Lösung gewaschen. Nach Trocknen und chromatographischer Reinigung wird die Zielverbindung erhalten.

**Beispiel 6A**

{2-(4-Bromphenyl)-8-fluor-3-[3-(trifluormethyl)phenyl]-3,4-dihydro-4-chinazolinyl}essigsäuremethylester

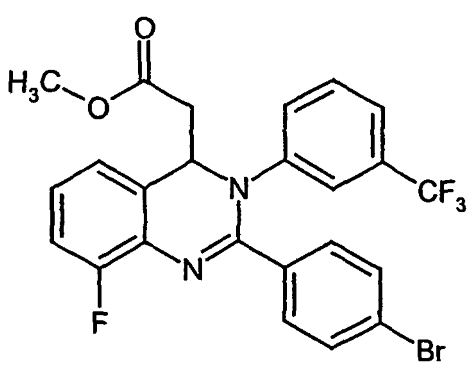

Ausgehend von 30.0 g (79.3 mmol) 2-Acylaminozimtsäureester aus Beispiel 3A werden nach der allgemeinen Arbeitsvorschrift [C-Variante 1] und nach Eluieren des Produktes von der Kieselgelsäule mit Cyclohexan, Cyclohexan / Ethylacetat 20:1, Cyclohexan / Ethylacetat 20:1.5, Cyclohexan / Ethylacetat 2:1, Cyclohexan / Ethylacetat 1:1, Ethylacetat sowie Methanol 39.3 g Produkt erhalten, das noch verunreinigt ist. Daher wird die Reinigung mittels Chromatographie an Kieselgel wiederholt, und es werden 11.5 g (55% d. Th.) Produkt erhalten.
HPLC (Methode 1): $R_t$= 4.66 min

**Beispiel 7A**

{2-(4-Brom-2-fluorphenyl)-8-fluor-3-[3-(triffuormethyl)phenyl]-3,4-dihydro-4-chinazolinyl}essigsäuremethylester

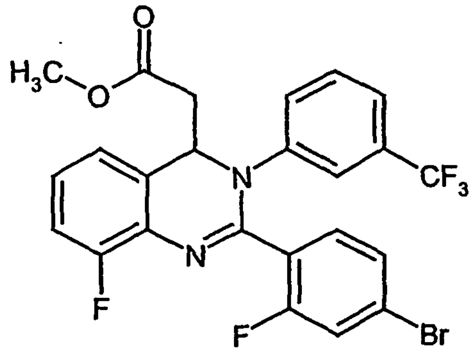

Ausgehend von 90 mg (0.23 mmol) 2-Acylaminozimtsäureester aus Beispiel 4A werden nach der allgemeinen Arbeitsvorschrift [C-Variante 1] 35 mg (29% d. Th.) Produkt erhalten.
HPLC (Methode 1): $R_t$= 4.66 min

**Beispiel 8A**

{2-(4-Bromphenyl)-8-fluor-3-[2-fluor-5-(trifluormethyl)phenyl]-3,4-dihydro-4-chinazolinyl}essigsäuremethylester

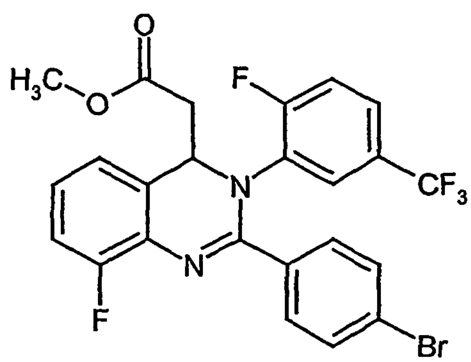

Ausgehend von 1.0 g (2.64 mmol) 2-Acylaminozimtsäureester aus Beispiel 3A werden nach der allgemeinen Arbeitsvorschrift [C-Variante 1] 394 mg (28% d. Th.) Produkt erhalten.
HPLC (Methode 1): $R_t$=4.71 min
MS (ESI-pos): m/z = 539 $(M+H)^+$

**Beispiel 9A**

{2-(4-Bromphenyl)-8-fluor-3-[4-fluor-3-(trifluormethyl)phenyl]-3,4-dihydro-4-chinazolinyl}essigsäuremethylester

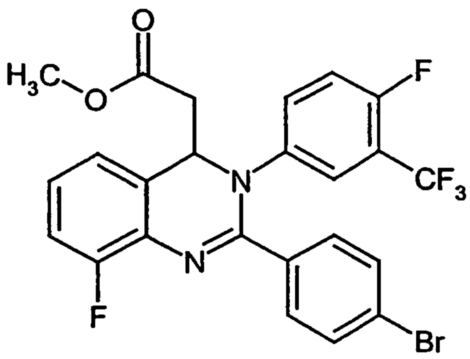

Ausgehend von 500 mg (1.32 mmol) 2-Acylaminozimtsäureester aus Beispiel 3A werden nach der allgemeinen Arbeitsvorschrift [C-Variante 1] 610 mg (68% d. Th.) Produkt erhalten.
HPLC (Methode 1): $R_t$= 4.70 min
MS (ESI-pos): m/z = 541 $(M+H)^+$

## Beispiel 10A

{2-(4-Bromphenyl)-8-fluor-6-methyl-3-[3-(trifluormethyl)phenyl]-3,4-dihydro-4-chinazolinyl}essigsäuremethylester

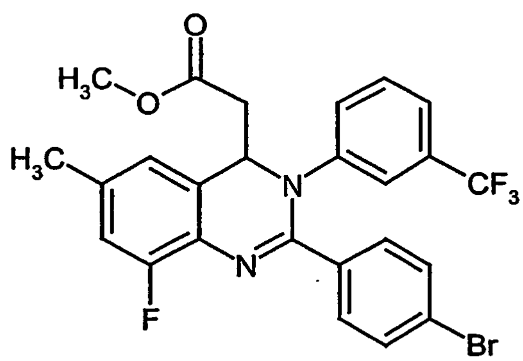

Ausgehend von 700 mg (1.79 mmol) 2-Acylaminozimtsäureester aus Beispiel 5A werden nach der allgemeinen Arbeitsvorschrift [C-Variante 1] 1.23 g (quantitativ) Produkt erhalten.
HPLC (Methode 1): $R_t$= 4.70 min
MS (ESI-pos): m/z = 535 $(M+H)^+$

## Beispiel 11A

{2-(4-Bromphenyl)-8-fluor-3-[2-methoxy-5-(trifluormethyl)phenyl]-3,4-dihydro-chinazolin-4-yl}essigsäuremethylester

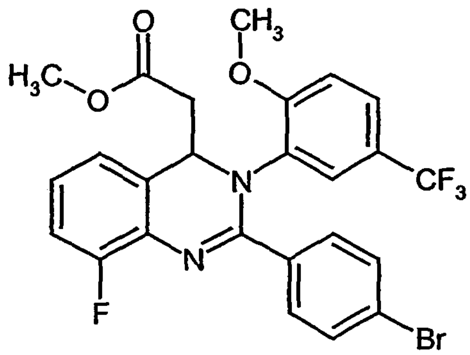

Ausgehend von 260 mg (0.47 mmol) 2-Acylaminozimtsäureester aus Beispiel 3A werden nach der allgemeinen Arbeitsvorschrift [C-Variante 3] 64 mg (23% d. Th.) Produkt erhalten.
HPLC (Methode 7): $R_t$= 2.97 min

**Beispiele 12A**

{2-(4-Bromphenyl)-8-fluor-3-(5-tert-butyl-2-methoxyphenyl)-3,4-dihydro-chinazolin-4-yl}essigsäuremethylester

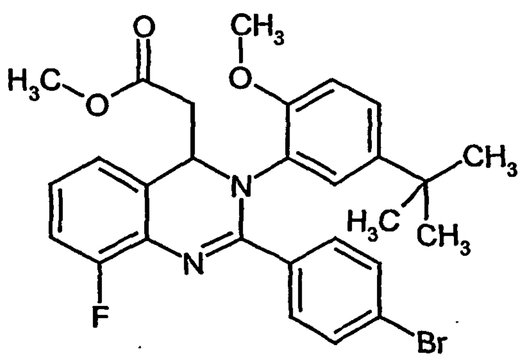

Ausgehend von 2500 mg (6.61 mmol) 2-Acylaminozimtsäureester aus Beispiel 5A werden nach der allgemeinen Arbeitsvorschrift [C-Variante 2] 417 mg (12% d. Th.) Produkt erhalten.
HPLC (Methode 5): $R_t$= 4.84 min
MS (ESI-pos): m/z = 539 $(M+H)^+$

**Beispiel 13A**

{2-(4-Bromphenyl)-8-fluor-3-[2-(trifluormethoxy)-5-(trifluormethyl)phenyl]-3,4-dihydro-chinazolin-4-yl}essigsäuremethylester

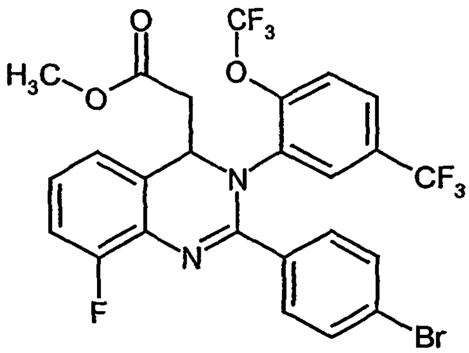

Ausgehend von 181 mg (0.46 mmol) 2-Acylaminozimtsäureester aus Beispiel 5A werden nach der allgemeinen Arbeitsvorschrift [C-Variante 2] 150 mg (53% d. Th.) Produkt erhalten.
HPLC (Methode 5): $R_t$= 5.02 min
MS (ESI-pos): m/z=621 $(M+H)^+$

**Allgemeine Arbeitsvorschrift [D]: Synthese der Biphenyle mittels SuzukiKupplung**

1.25 mmol Bromid, 1.50 mmol Boronsäure, 0.09 mmol Palladium(II)acetat 0.15 mmol Tris-cyclohexylphoshin und 1.5 mol Natriumcarbonat werden in 18 ml eines Dioxan-Wasser-Gemisches (5:1 v/v) vorgelegt und das Gemisch unter intensivem Rühren 16 h bei 80˚C erhitzt. Anschließend wird das Gemisch über eine Filterplatte filtriert, die Mutterlauge im Vakuum einrotiert und das Produkt mittels präparativer HPLC (Methode 4) oder durch Chromatographie an Kieselgel mit Cyclohexan/Ethylacetat - Gemischen gereinigt.

**Beispiel 14A**

{8-Fluor-2-(4'-fluor-1,1'-biphenyl-4-yl)-3-[3-(trifluormethyl)phenyl]-3,4-dihydro-4-chinazolinyl}essigsäuremethylester

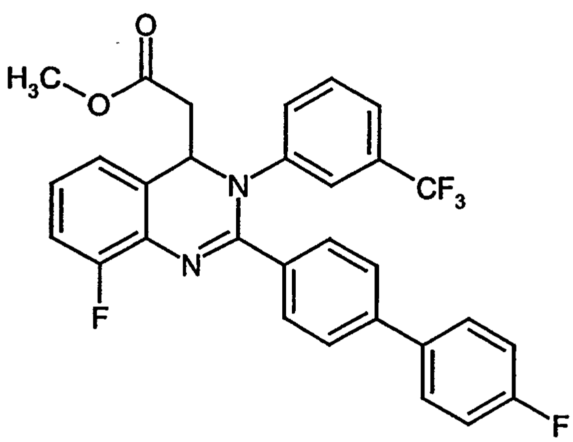

Ausgehend von 650 mg (1.25 mmol) des Bromids aus Beispiel 6A werden nach der allgemeinen Arbeitsvorschrift [D] und nach Reinigung mittels präparativer HPLC (Methode 4) 480 mg (72% d. Th.) Produkt erhalten.
HPLC (Methode 1): $R_t$= 4.87 min

**Beispiel 15A**

{8-Fluor-2-(3'-methyl-1,1'-biphenyl-4-yl)-3-[3-(trifluormethyl)phenyl]-3,4-dihydro-4-chinazolinyl} essigsäuremethylester

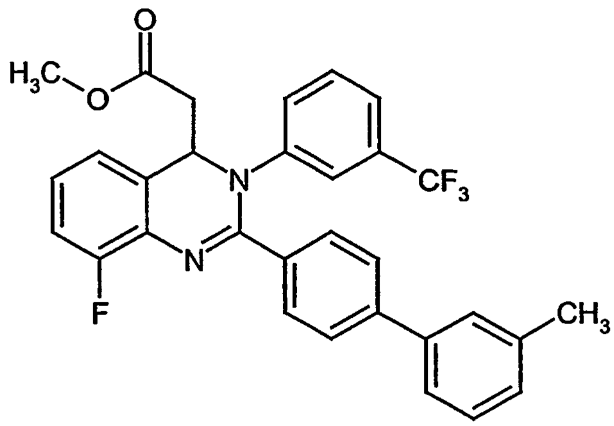

Ausgehend von 5.0 g (9.59 mmol) des Bromids aus Beispiel 6A werden nach der allgemeinen Arbeitsvorschrift [D] und nach Chromatographie an Kieselgel mit Cyclohexan/Ethylacetat 9:1 (v/v) und Cyclohexan/Ethylacetat 8:1 (v/v) 1.89 g (37% d. Th.) Produkt erhalten.
HPLC (Methode 1): $R_t$= 4.87 min

**Beispiel 16A**

{8-Fluor-2-(4'-fluor-3'-methyl-1,1'-biphenyl-4-yl)-3-[3-(trifluormethyl)phenyl]-3,4-dihydro-4-chinazolinyl}essigsäuremethylester

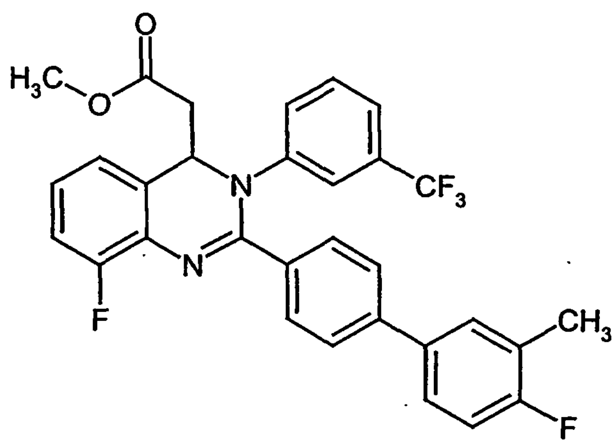

Ausgehend von 150 mg (0.29 mmol) des Bromids aus Beispiel 6A werden nach der allgemeinen Arbeitsvorschrift [D] und nach Reinigung mittels präparativer HPLC (Methode 4) 101 mg (64% d. Th.) Produkt erhalten.
HPLC (Methode 1): $R_t$= 4.94 min

**Beispiel 17A**

{8-Fluor-2-(3'-fluor-1,1'-biphenyl-4-yl)-3-[3-(trifluormethyl)phenyl]-3,4-dihydro-4-chinazolinyl}essigsäuremethylester

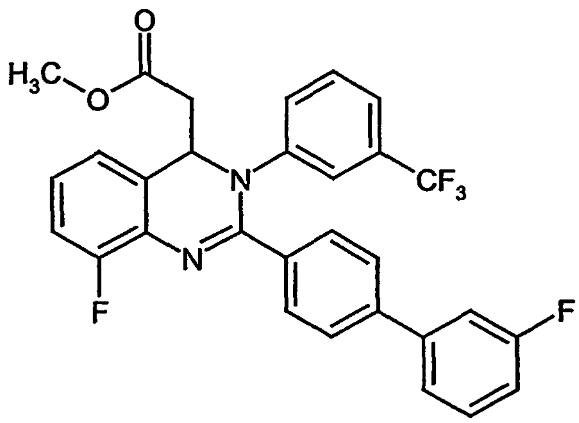

Ausgehend von 150 mg (1.25 mmol) des Bromids aus Beispiel 6A werden nach der allgemeinen Arbeitsvorschrift [D] und nach Reinigung mittels präparativer HPLC (Methode 4) 118 mg (76% d. Th.) Produkt erhalten.

**Beispiel 18A**

{2-(3,4'-Difluor-1,1'-biphenyl-4-yl)-8-fluor-3-[3-(trifluormethyl)phenyl]-3,4-dihydro-4-chinazolinyl}essigsäuremethylester

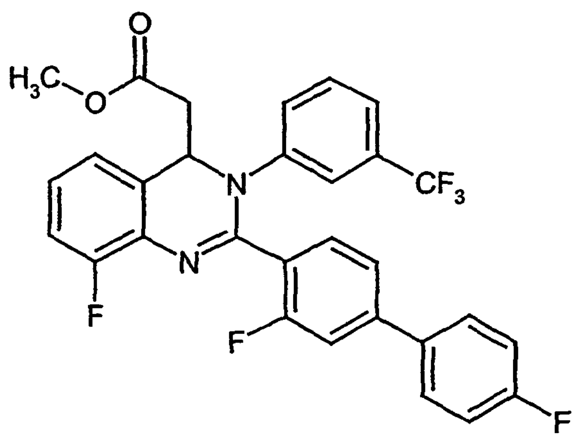

Ausgehend von 30 mg (0.06 mmol) des Bromids aus Beispiel 7A werden nach der allgemeinen Arbeitsvorschrift [D] und nach Reinigung mittels präparativer HPLC (Methode 4) 17 mg (55% d. Th.) Produkt erhalten.
HPLC (Methode 1): $R_t$= 4.83 min

**Beispiel 19A**

{2-[4-(1,3-Benzodioxol-5-yl)phenyl]-8-fluor-3-[2-methoxy-5-(trifluormethyl)-phenyl]-3,4-dihydro-4-chinazolinyl}essigsäuremethylester

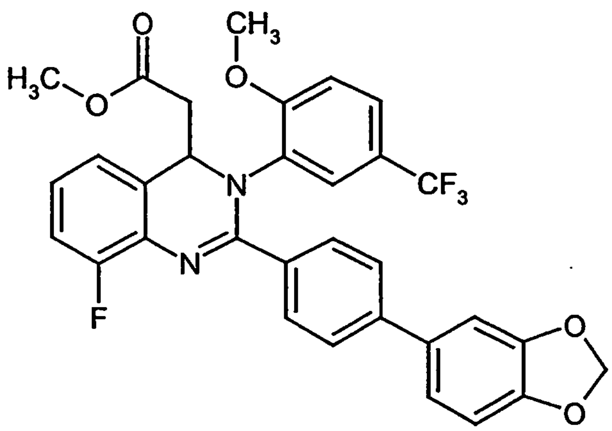

Ausgehend von 56 mg (0.09 mmol) des Bromids aus Beispiel 11A werden nach der allgemeinen Arbeitsvorschrift [D] und nach Reinigung mittels präparativer HPLC (Methode 4) 52 mg (92% d. Th.) Produkt erhalten.
HPLC (Methode 7): $R_t$= 2.70 min

**Beispiel 20A**

{2-[4-(4-Fluorphenyl)phenyl]-8-fluor-3-[2-methoxy-5-(trifluormethyl)-phenyl]-3,4-dihydro-4-chinazolinyl}essigsäuremethylester

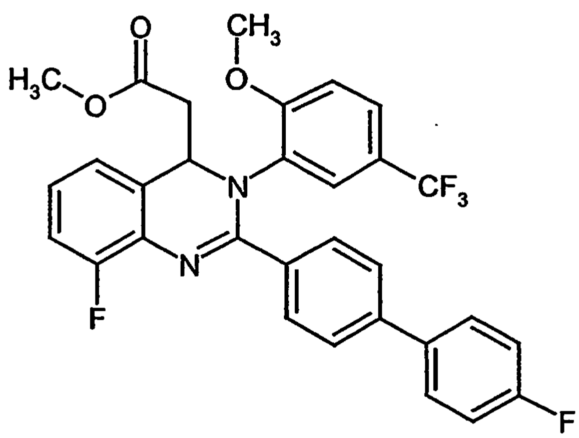

Ausgehend von 500 mg (0.91 mmol) des Bromids aus Beispiel 11A werden analog der allgemeinen Vorschrift [D] durch Umsetzung mit 152.27 mg (1.09 mmol) 4-Fluorphenylboronsäure, 31.83 mg (0.05 mmol) Bis(triphenylphosphin)-palladium-(II)chlorid und 115.34 mg (1.09 mmol) Natriumcarbonat in 10 ml 1,2-Dimethoxyethan und 0.5 ml Wasser 370.7 mg (72% d. Th.) der Zielverbindung erhalten.
HPLC (Methode 1): $R_t$= 4.8 min

MS (ESIpos): m/z = 567 $(M+H)^+$

**Beispiel 21A**

{2-[4-(3-Methylphenyl)phenyl]-8-fluor-3-[2-methoxy-5-(trifluorraethyl)-phenyl]-3,4-dihydro-4-chinazolinyl}essigsäure-methylester

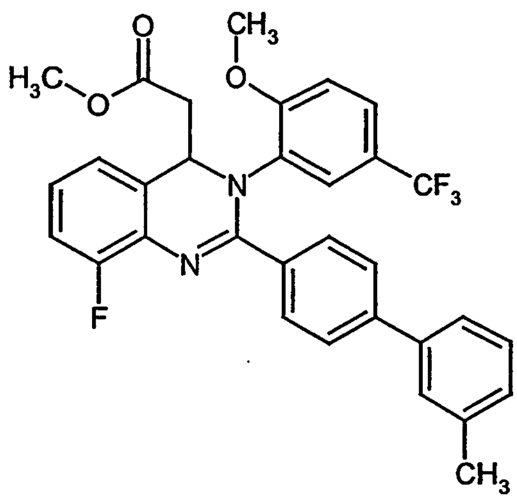

Ausgehend von 1.00 g (1.81 mmol) des Bromids aus Beispiel 11A werden analog der allgemeinen Vorschrift [D] durch Umsetzung mit 0.30 g (2.18 mmol) 3-Methylphenylboronsäure, 0.06 g (0.09 mmol) Bis(triphenylphosphin) palladium(II)chlorid und 0.23 g (2.18 mmol) Natriumcarbonat in 20 ml 1,2-Dimethoxyethan und 1 ml Wasser 450.5 mg (44% d. Th.) der Zielverbindung erhalten.
HPLC (Methode 1): $R_t$= 4.9 min
MS (ESIpos): m/z = 563 $(M+H)^+$

**Beispiel 22A**

{2-[4-(3-Methoxyphenyl)phenyl]-8-fluor-3-[2-methoxy-5-(trifluormethyl)phenyl]-3,4-dihydro-4-chinazolinyl}essigsäure-methylester

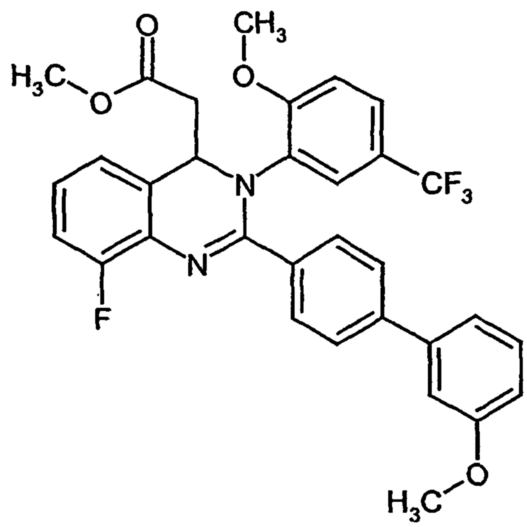

Ausgehend von 1.00 g (1.81 mmol) des Bromids aus Beispiel 11A werden nach der allgemeinen Vorschrift [D] durch Umsetzung mit 0.33 g (2.18 mmol) 3-Methoxyphenylboronsäure, 0.06 g (0.09 mmol) Bis(triphenylphosphin)palladium(II)chlorid und 0.23 g (2.18 mmol) Natriumcarbonat in 20 ml 1,2-Dimethoxyethan und 1 ml Wasser 466 mg (44% d. Th.) der Zielverbindung erhalten.
HPLC (Methode 1): $R_t$= 4.7 min
MS (ESIpos): m/z = 579 $(M+H)^+$

**Beispiel 23A**

{2-[4-(3-Fluorphenyl)phenyl]-8-fluor-3-[2-methoxy-5-(trifluormethyl)-phenyl]-3,4-dihydro-4-chinazolinyl}essigsäuremethylester

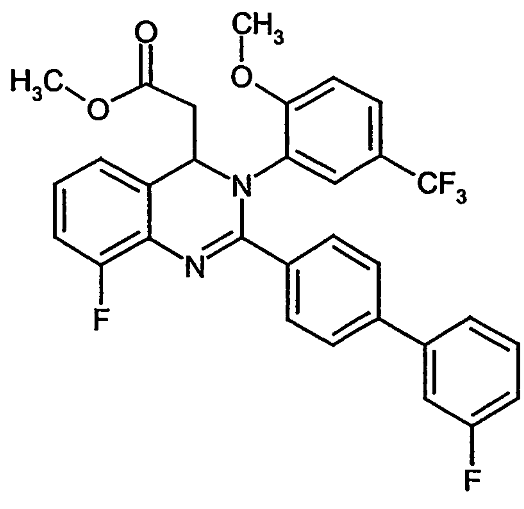

Ausgehend von 100 mg (0.18 mmol) des Bromids aus Beispiel 11A werden nach der allgemeinen Vorschrift [D] durch Umsetzung mit 30.5 mg (0.22 mmol) 3-Fluorphenylboronsäure, 6.37 mg (0.01 mmol) Bis(triphenylphosphin) palladium(II)chlorid und 23.07 mg (0.22 mmol) Natriumcarbonat in 10 ml 1,2-Dimethyoxyethan und 0.1 ml Wasser 22.6 mg (22% d. Th.) der Zielverbindung erhalten.

HPLC (Methode 5): $R_t$= 4.9 min
MS (ESIpos): m/z = 567 $(M+H)^+$

Die Beispiele 24A bis 44A der Tabelle 1 werden analog der allgemeinen Vorschrift [D] hergestellt.

**Tabelle** 1

| Beispiel | Struktur | Molekulargewicht | HPLC $R_t$ [min] | HPLC/LC MS-Methode | m/z $(M+H)^+$ |
|---|---|---|---|---|---|
| 24A | 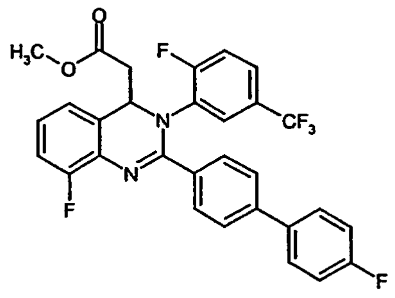 | 554.5 | 4.81 | 1 | 555 |
| 25A | 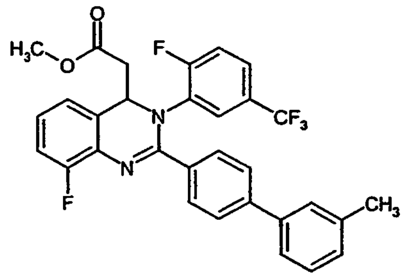 | 550.5 | 4.92 | 1 | 551 |
| 26A | 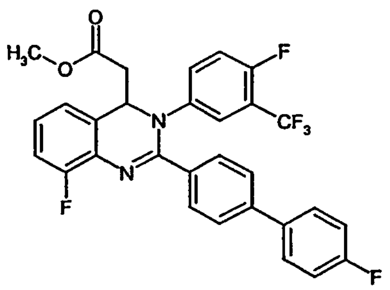 | 554.5 | 4.30 | 3 | 555 |
| 27A | 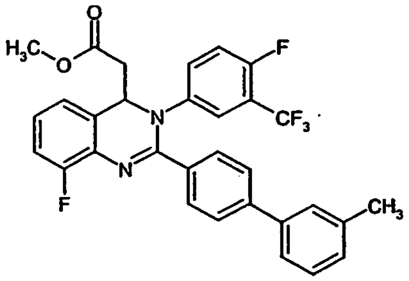 | 550.5 | 5.00 | 1 | 551 |

(fortgesetzt)

| Beispiel | Struktur | Molekulargewicht | HPLC $R_t$ [min] | HPLC/LC MS-Methode | m/z $(M+H)^+$ |
|---|---|---|---|---|---|
| 28A | 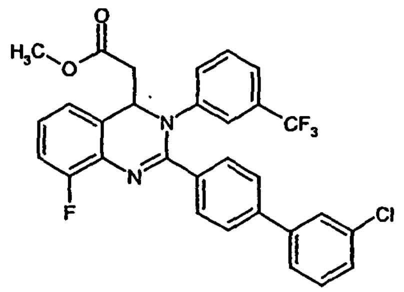 | 552.9 | 4.90 | 1 | 553 |
| 29A | 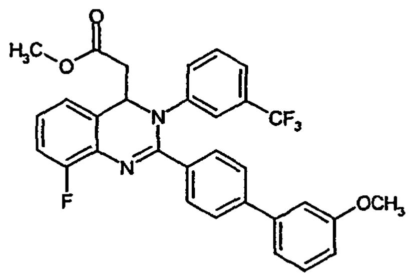 | 548.5 | 4.75 | 1 | 549 |
| 30A | 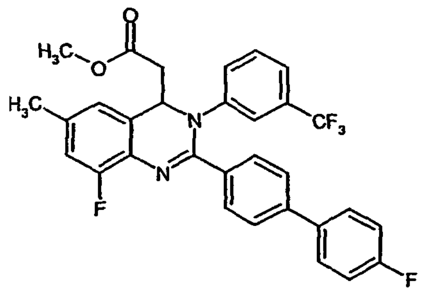 | 550.5 | 4.91 | 1 | 551 |
| 31A | 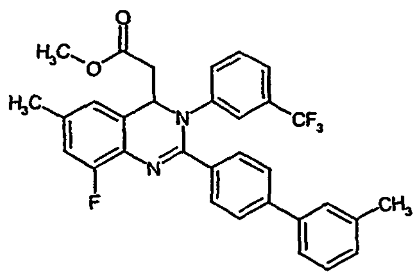 | 546.5 | 5.03 | 1 | 547 |
| 32A | 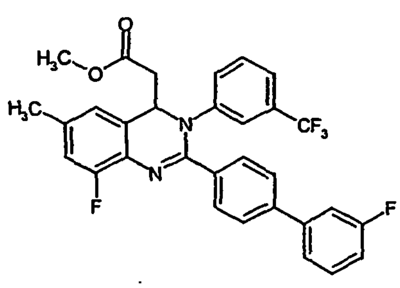 | 550.5 | 4.91 | 1 | 550 |

(fortgesetzt)

| Beispiel | Struktur | Molekulargewicht | HPLC $R_t$ [min] | HPLC/LC MS-Methode | m/z $(M+H)^+$ |
|---|---|---|---|---|---|
| 33A | 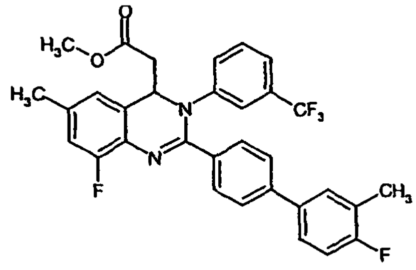 | 564.5 | 5.04 | 1 | 565 |
| 34A | 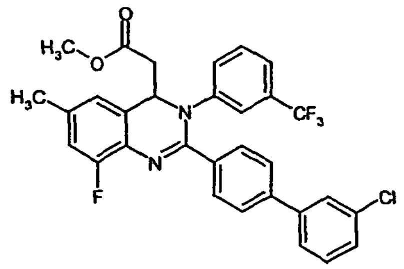 | 567 | 5.11 | 1 | 567 |
| 35A | 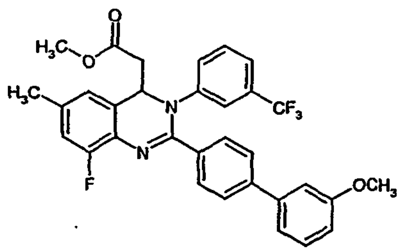 | 562.5 | 4.89 | 1 | 563 |
| 36A | 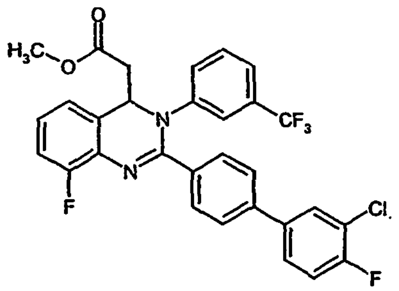 | 570.9 | 5.03 | 1 | 571 |
| 37A | 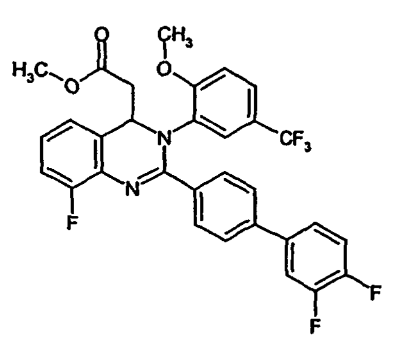 | 584.5 | 4.80 | 1 | 585 |

(fortgesetzt)

| Beispiel | Struktur | Molekulargewicht | HPLC $R_t$ [min] | HPLC/LC MS-Methode | m/z $(M+H)^+$ |
|---|---|---|---|---|---|
| 38A | 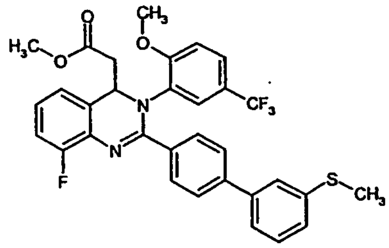 | 594.6 | 3.99 | 7 | 595 |
| 39A | 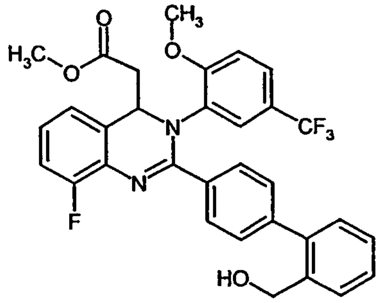 | 578.5 | 4.55 | 5 | 579 |
| 40A | 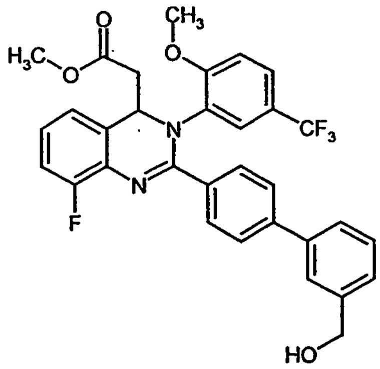 | 578.5 | 3.93 | 8 | 579 |
| 41A | 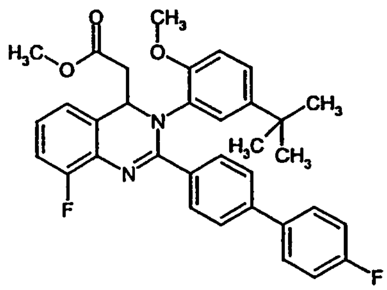 | 554.6 | 5.09 | 5 | 555 |

(fortgesetzt)

| Beispiel | Struktur | Molekulargewicht | HPLC $R_t$ [min] | HPLC/LC MS-Methode | m/z $(M+H)^+$ |
|---|---|---|---|---|---|
| 42A | 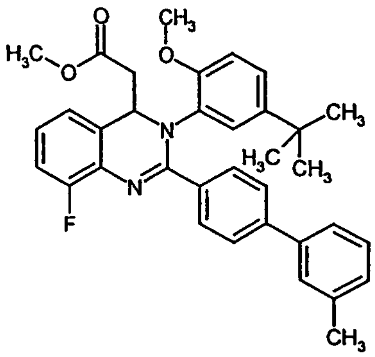 | 550.6 | 5.22 | 5 | 551 |
| 43A | 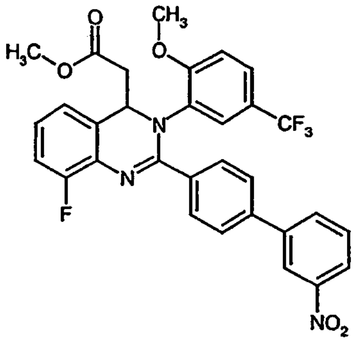 | 593.5 | 2.97 | 8 | 594 |
| 44A | 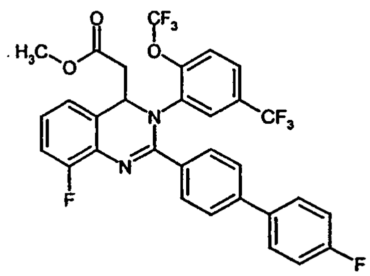 | 620.5 | 5.02 | 5 | 621 |

## Ausführungsbeispiele

### Allgemeine Arbeitsvorschrift [E]: Esterverseifung der Chinazolylessigsäurester

Es werden 1.0 Äquivalente des Chinazolylessigsäuresters in Dioxan gelöst und 5.0 Äquivalente 1N Natronlauge hinzugefügt. Man lässt für 2 Stunden bei 50°C rühren und nach beendeter Reaktion (Reaktionskontrolle mittels analytischer HPLC) wird der Ansatz eingeengt. Der Rückstand wird dann in Wasser aufgenommen und mit 1N Salzsäure auf pH = 5 gestellt. Man filtriert den entstehenden Niederschlag ab, wäscht ihn mit wenig Wasser und Diethylether und trocknet ihn im Hochvakuum bei Raumtemperatur. Alternativ kann der Niederschlag über eine Extrelutkartusche filtriert, mit Ethylacetat nachgewaschen und das Filtrat eingeengt werden. Falls die Reinheit des Produktes nicht hoch genug ist, wird es über präparative HPLC an RP-Phase gereinigt (Methode 4).

**Beispiel 1**

{8-Fluor-2-(4'-ffuor-1,1'-biphenyl-4-yl)-3-[3-(trifluormethyl)phenyl]-3,4-dihydro-4-chinazolinyl}essigsäure

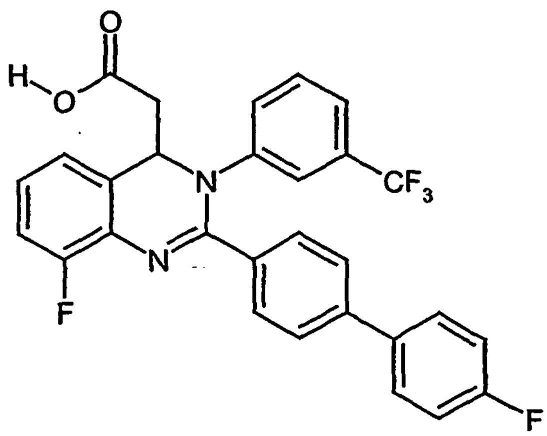

Ausgehend von 1.13 g (2.1 mmol) Methylester aus Beispiel 14A werden nach der allgemeinen Arbeitsvorschrift [E] 1.10 g (97% d. Th.) Produkt erhalten.
HPLC (Methode 1): $R_t$= 4.64 min
MS (ESIpos): m/z = 523.4 $(M+H)^+$
$^1$H-NMR (200MHz, $CD_3CN$): δ [ppm] = 7.83 (d, 2H); 7.68-7.57 (m, 5H); 7.33 (d, 2H); 7.28-7.19 (m, 5H); 7.05-7.01 (m, 1H); 5.55-5.47 (m, 1H); 2.87 (dd, 1H); 2.66 (dd, 1H).

**Beispiel 2**

{8-Fluor-2-(4'-fluor-1,1'-biphenyl-4-yl)-3-[3-(trifluormethyl)phenyl]-3,4-dihydro-4-chinazolinyl}essigsäure

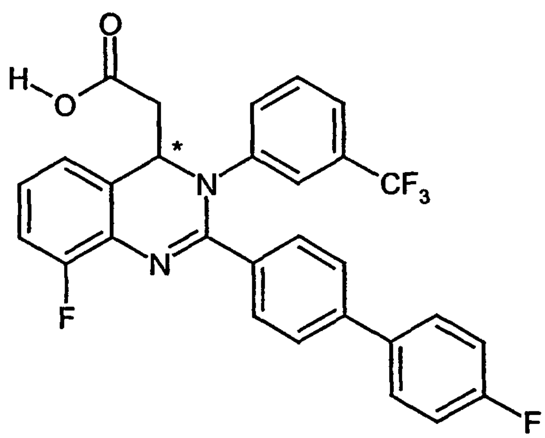

Durch Trennung von 359 mg des Racemats aus Beispiel 1 nach Methode 2 werden 118 mg (66% d. Th.) Produkt erhalten.
HPLC (Methode 1): $R_t$= 4.62 min
MS (ESIpos): m/z = 522.9 $(M+H)^+$
$^1$H-NMR (400MHz, $CD_3CN$): δ [ppm] = 7.84 (d, 2H); 7.82 (d, 2H); 7.65-7.57 (m, 5H); 7.33-7.31 (m, 2H); 7.23-7.13 (m,

5H); 7.02 (d, 1H); 5.50 (t, 1H); 2.86 (dd, 1H); 2.67 (dd, 1H).

**Beispiel 3**

{8-Fluor-2-(3'-methyl-1,1'-biphenyl-4-yl)-3-[3-(trifluormethyl)phenyl]-3,4-dihydro-4-chinazolinyl}essigsäure

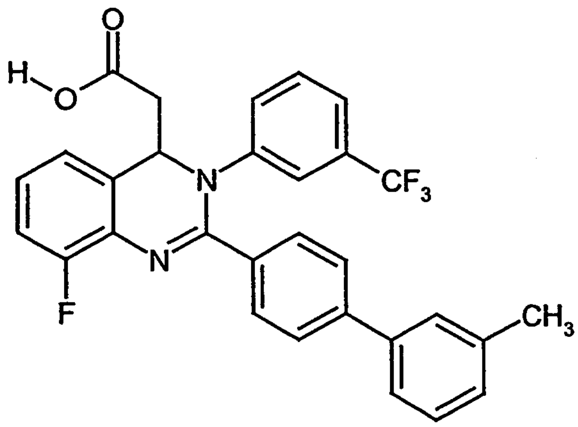

Ausgehend von 1.13 g (2.1 mmol) Methylester aus Beispiel 15A werden nach der allgemeinen Arbeitsvorschrift [E] 1.10 g (97% d. Th.) Produkt erhalten.
HPLC (Methode 1): $R_t$= 4.74 min
MS (ESIpos): m/z = 519 $(M+H)^+$
$^1$H-NMR (400MHz, $CD_3CN$): δ [ppm] = 7.83 (s, 2H); 7.59 (s, 1H); 7.49 (d, 2H); 7.35-7.24 (m, 6H); 7.18-7.09 (m, 3H); 7.00 (d, 1H); 5.51 (t, 1H); 2.81-2.75 (m, 1H); 2.60-2.55 (m, 1H); Signal für $CH_3$-Gruppe unter Lösungsmittelsignal.

**Beispiel 4**

{8-Fluor-2-(3'-methyl-1,1'-biphenyl-4-yl)-3-[3-(trifluormethyl)phenyl]-3,4-dihydro-4-chinazolinyl}essigsäure

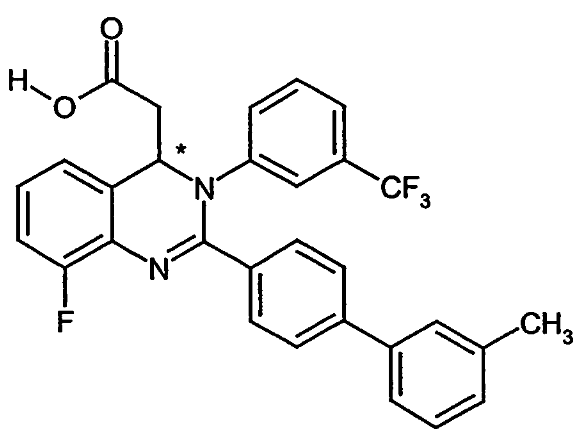

Durch Trennung von 2.48 g des Racemats aus Beispiel 3 nach Methode 2 und erneuter Reinigung mittels präparativer HPLC (Methode 4) werden 449 mg (36 % d. Th.) Produkt erhalten.
HPLC (Methode 1): $R_t$= 4.72 min
MS (ESIpos): m/z = 518.8 $(M+H)^+$

$^1H$-NMR (400MHz, $CD_3CN$): δ [ppm] = 7.83 (d, 2H); 7.61-7.59 (m, 3H); 7.45 (s, 1H); 7.41 (d, 1H); 7.35-7.31 (m, 3H); 7.24-7.14 (m, 4H); 7.02 (d, 1H); 5.50 (dd, 1H); 2.86 (dd, 1H); 2.67 (dd, 1H); 2.38 (s 3H).

**Beispiel 5**

{8-Fluor-2-(4'-fluor-3'-methyl-1,1'-biphenyl-4-yl)-3-[3-(trifluornlethyl)phenyl]-3,4-dihydro-4-chinazolinyl}essigsäure

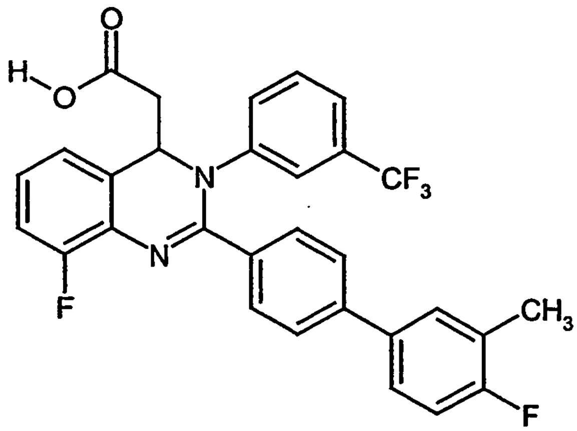

Ausgehend von 80 mg (0.15 mmol) Methylester aus Beispiel 16A werden nach der allgemeinen Arbeitsvorschrift [E] 36 mg (46% d. Th.) Produkt erhalten.
HPLC (Methode 1): $R_t$= 4.75 min
MS (ESIpos): m/z = 537 $(M+H)^+$
$^1H$-NMR (300MHz, $CD_3CN$): δ [ppm] = 7.84-7.79 (m, 2H); 7.59-7.41 (m, 5H); 7.33-7.31 (m, 2H); 7.25-7.01 (m, 5H); 5.52-5.47 (m, 1H); 2.86 (dd, 1H); 2.68 (dd, 1H); 2.30 (s, 3H).

**Beispiel 6**

{8-Fluor-2-(3'-fluor-1,1'-biphenyl-4-yl)-3-[3-(trifluormethyl)phenyl]-3,4-dihydro-4-chinazolinyl}essigsäure

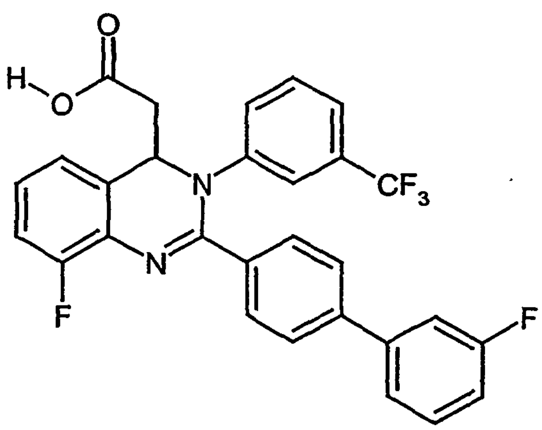

Ausgehend von 80 mg (0.15 mmol) Methylester aus Beispiel 17A werden nach der allgemeinen Arbeitsvorschrift [E] 75 mg (95% d. Th.) Produkt erhalten.
HPLC (Methode 1): $R_t$=4.61 min

MS (ESIpos): m/z = 523 (M+H)+
$^1$H-NMR (300MHz, $CD_3CN$): δ [ppm] = 7.87-7.83 (m, 2H); 7.64-7.59 (m, 3H); 7.48-7.29 (m, 5H); 7.25-7.09 (m, 4H); 7.04-7.01 (m, 1H); 5.52-5.47 (m, 1H); 2.86 (dd, 1H); 2:68 (dd, 1H).

**Beispiel 7**

{2-(3,4'-Difluor-1,1'-biphenyl-4-yl)-8-fluor-3-[3-(trifluormethyl)phenyl]-3,4-dihydro-4-chinazolinyl}essigsäure

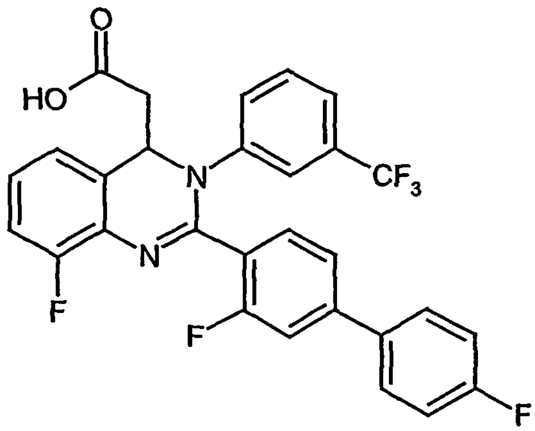

Ausgehend von 15 mg (0.03 mmol) Methylester aus Beispiel 18A werden nach der allgemeinen Arbeitsvorschrift [E] 14 mg (96% d. Th.) Produkt erhalten.
HPLC (Methode 1): $R_t$ 4.65 min
MS (ESIpos): m/z = S41 (M+H)+
$^1$H-NMR (400MHz, $CD_3CN$): δ [ppm] = 2.77 (dd, 1H); 3.02 (dd, 1H); 5.51 (t, 1H); 7.02 (d, 1H); 7.10-7.25 (m, 5H); 7.37 (s, 3H), 7.47-7.55 (m, 2H); 7.57-7.67 (m, 2H); 7.86 (dd, 1H).

Die Beispiele 8 bis 29 der Tabelle 2 werden analog der allgemeinen Vorschrift [E] hergestellt.

**Tabelle 2**

| Beispiel | Struktur | Molekulargewicht | HPLC $R_t$ [min] | HPLC/ LCMS-Methode | m/z $(M+H)^+$ |
|---|---|---|---|---|---|
| 8 | 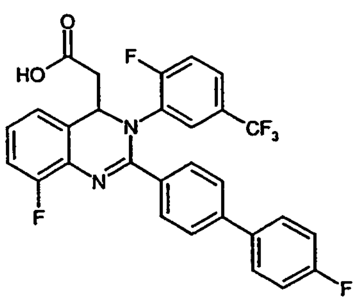 | 540.5 | 4.0 | 3 | 541 |

(fortgesetzt)

| Beispiel | Struktur | Molekulargewicht | HPLC $R_t$ [min] | HPLC/ LCMS-Methode | m/z $(M+H)^+$ |
|---|---|---|---|---|---|
| 9 | 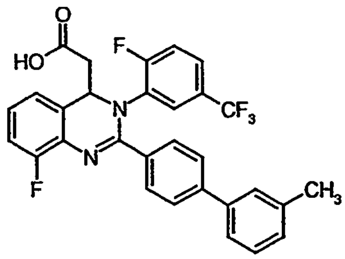 | 536.5 | 4.1 | 3 | 537 |
| 10 | 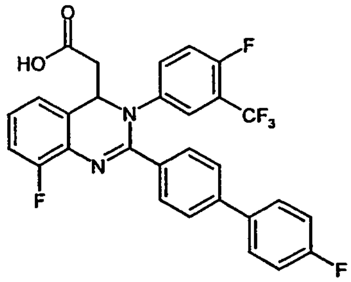 | 540.5 | 4.0 | 3 | 541 |
| 11 | 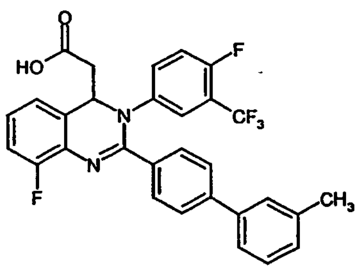 | 536.5 | 4.1 | 3 | 537 |
| 12 | 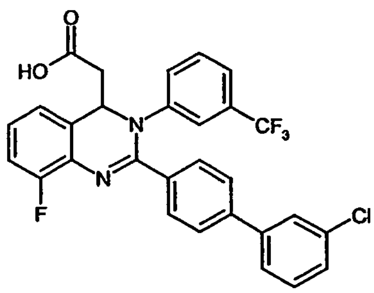 | 538.9 | 4.81 | 1 | 539 |
| 13 | 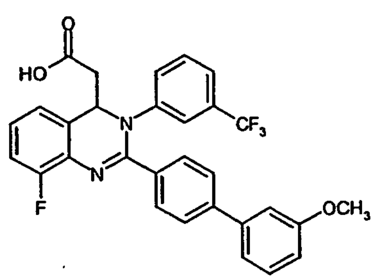 | 534.5 | 4.66 | 1 | 535 |

(fortgesetzt)

| Beispiel | Struktur | Molekulargewicht | HPLC $R_t$ [min] | HPLC/ LCMS-Methode | m/z $(M+H)^+$ |
|---|---|---|---|---|---|
| 14 | 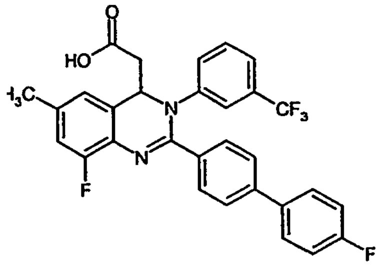 | 536.5 | 4.61 | 1 | 537 |
| 15 | 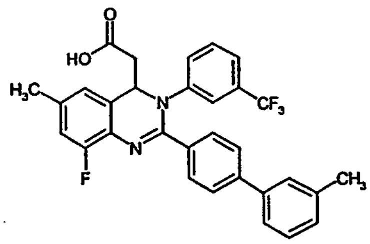 | 532.5 | 4.71 | 1 | 533 |
| 16 | 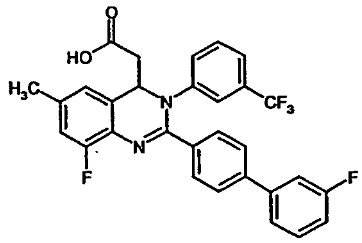 | 536.5 | 4.61 | 1 | 537 |
| 17 | 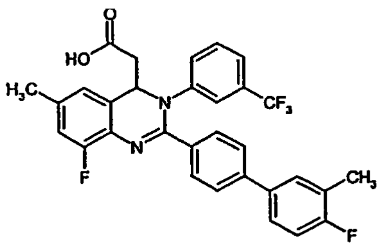 | 550.5 | 4.73 | 1 | 551 |
| 18 | 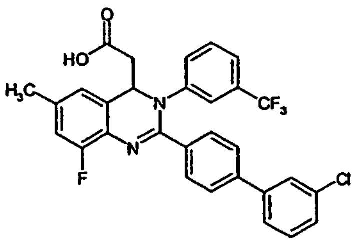 | 553.0 | 4.72 | 1 | 553 |

(fortgesetzt)

| Beispiel | Struktur | Molekulargewicht | HPLC $R_t$ [min] | HPLC/ LCMS-Methode | m/z $(M+H)^+$ |
|---|---|---|---|---|---|
| 19 | 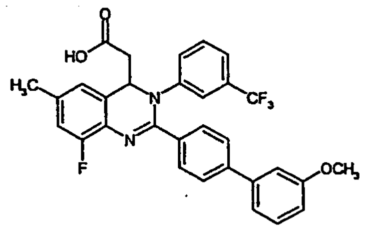 | 548.5 | 4.6 | 1 | 549 |
| 20 | 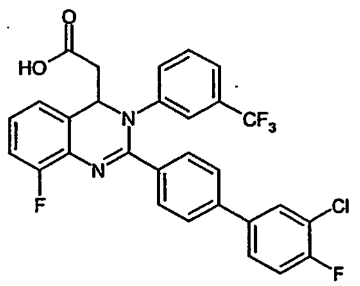 | 556.9 | 4.75 | 1 | 557 |
| 21 | 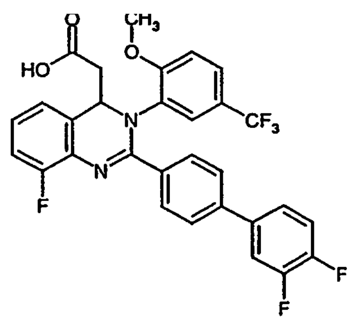 | 570.5 | 4.6 | 1 | 571 |
| 22 | 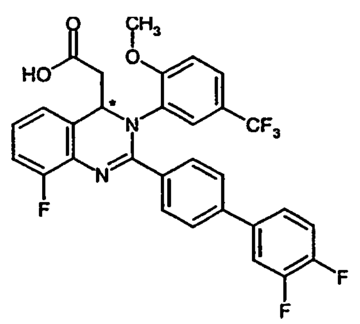 | 570.5 | 4.6 | 5 | 571 |
| 23 | 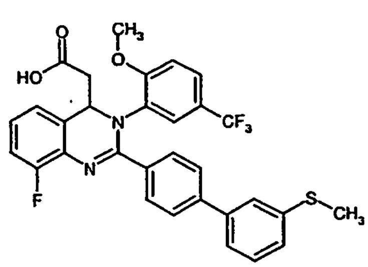 | 580.6 | 3.1 | 7 | 581 |

(fortgesetzt)

| Beispiel | Struktur | Molekulargewicht | HPLC $R_t$ [min] | HPLC/ LCMS-Methode | m/z $(M+H)^+$ |
|---|---|---|---|---|---|
| 24 | 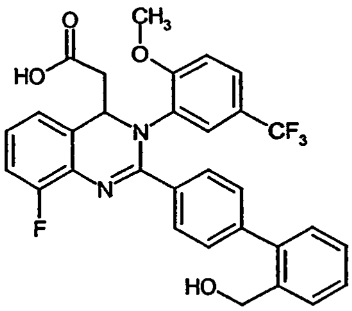 | 564.5 | 3.8 | 8 | 565 |
| 25 | 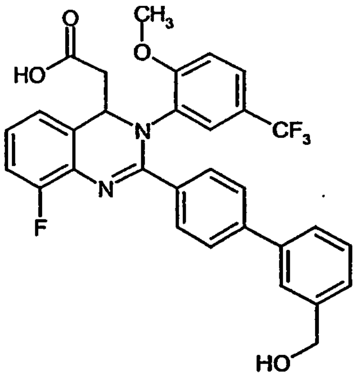 | 564.5 | 2.4 | 3 | 565 |
| 26 | 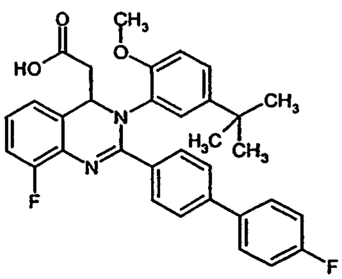 | 540.6 | 2.8 | 7 | 541 |
| 27 | 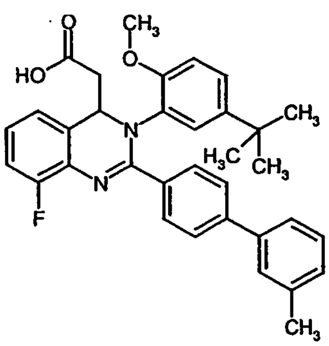 | 536.6 | 5.0 | 1 | 537 |

(fortgesetzt)

| Beispiel | Struktur | Molekulargewicht | HPLC $R_t$ [min] | HPLC/ LCMS-Methode | m/z $(M+H)^+$ |
|---|---|---|---|---|---|
| 28 | 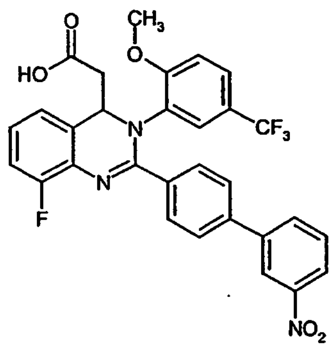 | 579.5 | 3.2 | 7 | 580 |
| 29 | 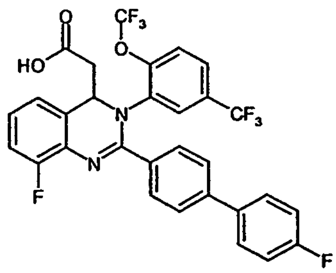 | 606.5 | 4.9 | 1 | 607 |

**Beispiel 30**

{2-[4-(1,3-Benzodioxol-5-yl)phenyl]-8-fluor-3-[2-methoxy-5-(trifluormethyl)-phenyl]-3,4-dihydro-4-chinazolinyl} essigsäure

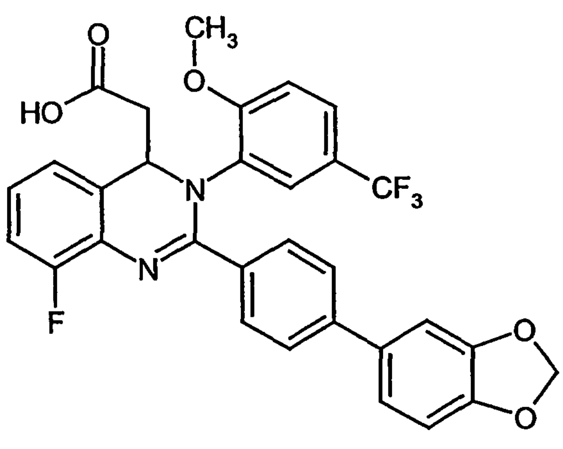

Ausgehend von 56 mg (0.09 mmol) Methylester aus Beispiel 19A werden nach der allgemeinen Arbeitsvorschrift [E] 52 mg (92% d. Th.) Produkt erhalten.

HPLC (Methode 7): $R_t$= 3.13 min

MS (ESIpos): m/z = 579 $(M+H)^+$

$^1$H-NMR (400MHz, $CD_3CN$): δ [ppm] = 7.70 (d, 1H); 7.57 (d, 1H); 7.50-7.40 (m, 4H), 7.14-7.08 (m, 4H); 6.94-6.88 (m, 2H); 5.13 (dd, 1H); 3.60 (s, 3H); 3.05-3.00 (m, 2H); 2.62 (dd, 2H).

**Beispiel 31**

{2-[4-(4-Fluorphenyl)phenyl]-8-fluor-3-[2-methoxy-5-(trifluormethyl)phenyl]-3,4-dihydro-4-chinazolinyl} essigsäure

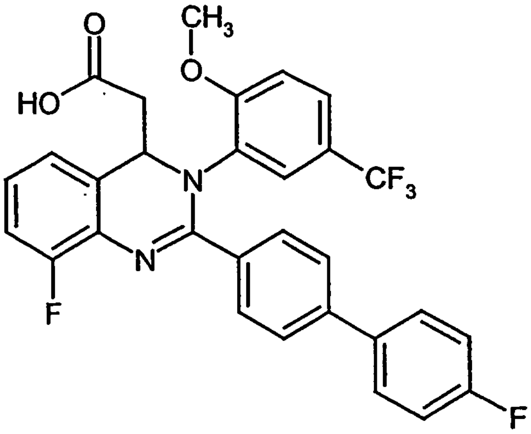

Ausgehend von 38.90 mg (0.07 mmol) des Esters aus Beispiel 20A werden analog der allgemeinen Arbeitsvorschrift [E] durch Umsetzung mit 8.24 mg (0.21 mmol) Natriumhydroxid in 10 ml Dioxan 29 mg (76% d. Th.) der Zielverbindung erhalten.
HPLC (Methode 1): $R_t$ = 4.6 min
MS (ESIpos): m/z = 553 $(M+H)^+$
$^1$H-NMR (200 MHz, DMSO-$d_6$): δ [ppm] = 2.55-2.63 (m, 2H); 3.62 (s, 3H); 5.13-5.30 (m, 1H); 6.90- 7.80 (m, 14H); 12.5-12.8 (br.s, 1H).

**Beispiel 32**

{2-[4-(4-Fluorphenyl)phenyl]-8-fluor-3-[2-methoxy-5-(trifluormethyl)-phenyl]-3,4-dihydro-4-chinazolinyl}essigsäure

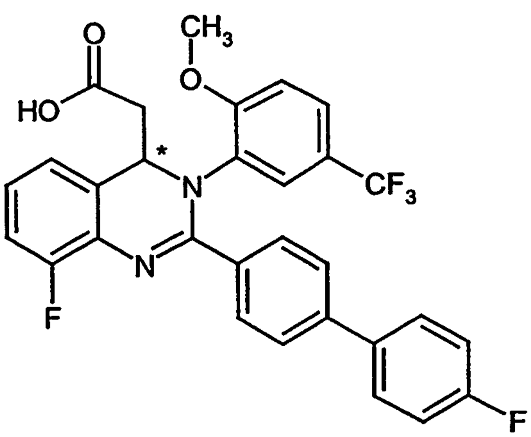

Durch Trennung von 172 mg des Racemats aus Beispiel 31 nach Methode 9 und erneuter Reinigung mittels Chromatographie an Kieselgel (Dichlormethan, Dichlormethan/Methanol 10:1) werden 60 mg (35% d. Th.) des Produkts erhalten.

HPLC (Methode 5): $R_t$ = 4.6 min
MS (ESIpos): m/z = 553 $(M+H)^+$
$^1$H-NMR (200 MHz, DMSO-$d_6$): δ [ppm] = 2.60-2.90 (br.m, 2H); 3.10-3.80 (br.s, 3H); 5.00-5.20 (m, 1H); 6.80- 7.80 (m, 14H).

**Beispiel 33**

{2-[4-(3-Methylphenyl)phenyl]-8-fluor-3-[2-methoxy-5-(trifluormethyl)-phenyl]-3,4-dihydro-4-chinazolinyl} essigsäure

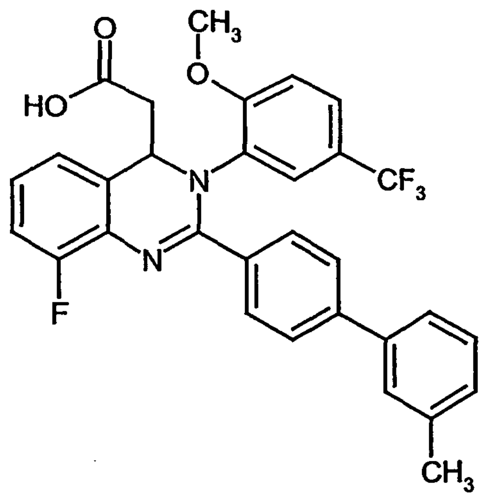

Ausgehend von 450 mg (0.80 mmol) des Esters aus Beispiel 21A werden analog der allgemeinen Arbeitsvorschrift [E] durch Umsetzung mit 96 mg (2.40 mmol) Natriumhydroxid in 21.5 ml Dioxan 407.5 mg (93% d. Th.) der Zielverbindung erhalten.
HPLC (Methode 1): $R_t$ = 4.7 min
MS (ESIpos): m/z = 549 $(M+H)^+$

**Beispiel 34**

{2-[4-(3-Methylphenyl)phenyl]-8-fluor-3-[2-methoxy-5-(trifluormethyl)-phenyl]-3,4-dihydro-4-chinazolinyl} essigsäure

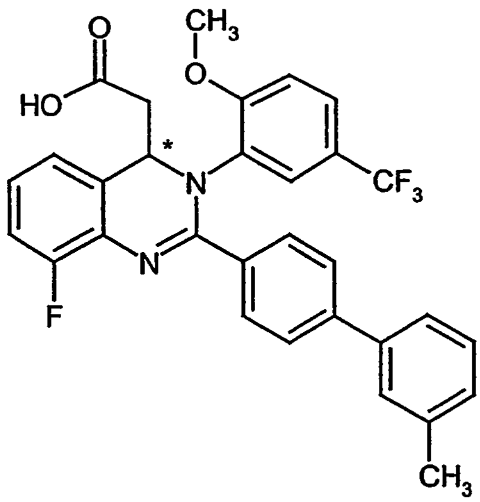

Durch Trennung von 407.5 mg des Racemats aus Beispiel 33 nach Methode 10 und erneuter Reinigung mittels Kristallisation aus Diethylether werden 153.6 mg (38% d. Th.) des Produkts erhalten.
HPLC (Methode 5): $R_t$ = 4.7 min
MS (ESIpos): m/z = 549 $(M+H)^+$
$^1$H-NMR (300 MHz, DMSO-$d_6$): δ [ppm] = 2.35 (s, 3H), 2.50-2.60 (m, 1H); 2.90-3.10 (m, 1H), 3.40-3.80 (br.s, 3H); 5.10-5.20 (m, 1H); 6.90-7.75 (m, 14H); 12.5-12.7 (br.s, 1H).

**Beispiel 35**

{2-[4-(3-Methoxyphenyl)phenyl]-8-fluor-3-[2-methoxy-5-(trifluormethyl)-phenyl]-3,4-dihydro-4-chinazolinyl} essigsäure

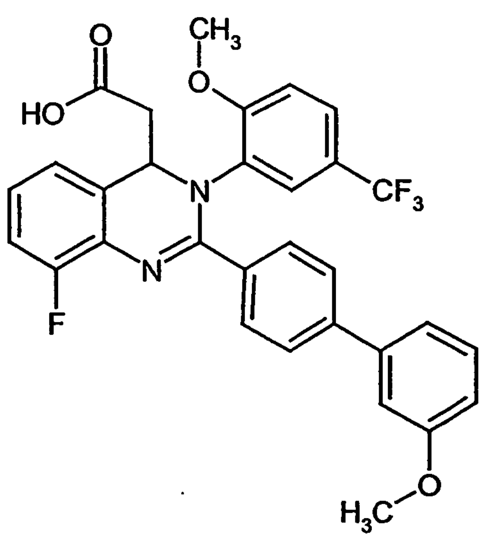

Ausgehend von 459 ing (0.79 mmol) des Esters aus Beispiel 22A werden analog der allgemeinen Arbeitsvorschrift [E] durch Umsetzung mit 95.2 mg (2.38 mmol) Natriumhydroxid in 54 ml Dioxan 383 mg (86% d. Th.) der Zielverbindung erhalten.
HPLC (Methode 1): $R_t$ = 4.5 min
MS (ESIpos): m/z = 565 $(M+H)^+$

**Beispiel 36**

{2-[4-(3-Methoxyphenyl)phenyl]-8-fluor-3-[2-methoxy-5-(trifluormethyl)-phenyl]-3,4-dihydro-4-chinazolinyl} essigsäure

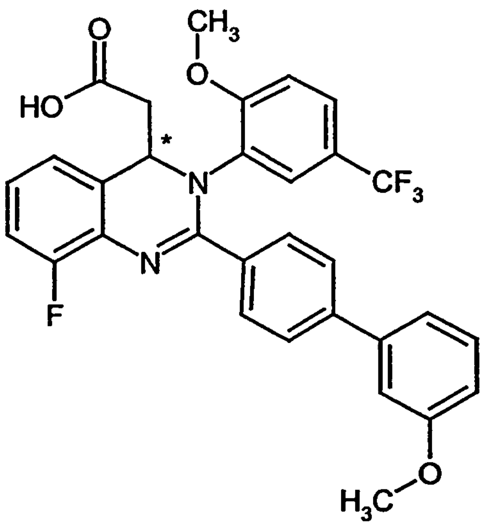

Durch Trennung von 46 mg des Racemats aus Beispiel 35 nach Methode 6 und erneuter Reinigung mittels Kristallisation aus Diethylether werden 13 mg (28% d. Th.) des Produkts erhalten.
HPLC (Methode 5): $R_t$ = 4.6 min
MS (ESIpos): m/z = 565 $(M+H)^+$
$^1$H-NMR (300 MHz, DMSO-$d_6$): δ [ppm] = 2.50-2.60 (m, 1H); 2.90-3.10 (m, 1H), 3.30 (s, 3H); 3.80 (s, 3H); 5.10-5.20 (m, 1H); 6.90-7.75 (m, 14H); 12.5-12.7 (br.s, 1H).

**Beispiel 37**

{2-[4-(3-Fluorphenyl)phenyl]-8-fluor-3-[2-methoxy-5-(trifluormethyl)-phenyl]-3,4-dihydro-4-chinazolinyl} essigsäure

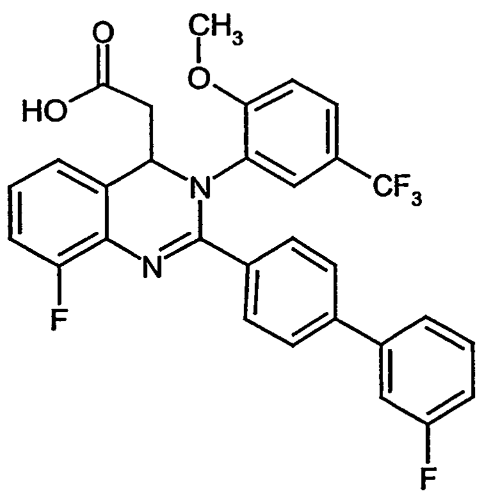

Ausgehend von 20.00 mg (0.04 mmol) des Esters aus Beispiel 23A werden analog der allgemeinen Arbeitsvorschrift [E] durch Umsetzung mit 4.24 mg (0.11 mmol) Natriumhydroxid in 10 ml Dioxan 18.7 mg (96% d. Th.) der Zielverbindung erhalten.
HPLC (Methode 5): $R_t$ = 4.6 min
MS (ESIpos): m/z = 553 $(M+H)^+$
$^1$H-NMR (300 MHz, DMSO-$d_6$): $\delta$ [ppm] = 2.50-2.65 (m, 1H); 2.90-3.10 (m, 1H), 3.50-3.7 (br.s, 3H); 3.80 (s, 3H); 5.10-5.20 (m, 1H); 6.90-7.75 (m, 14H), 12.50-12.60 (br.s, 1H).

**B. Bewertung der physiologischen Wirksamkeit**

Die in vitro-Wirkung der erfindungsgemäßen Verbindungen kann in folgenden Assays gezeigt werden:

**Anti-HCMV- (Anti-Humanes Cytomegalo-Virus) Zytopathogenitätstests**

Die Testverbindungen werden als 50 millimolare (mM) Lösungen in Dimethylsulfoxid (DMSO) eingesetzt. Ganciclovir, Foscarnet und Cidofovir dienen als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x $10^4$ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0,001 - 0,002), d.h. 1-2 infizierte Zellen auf 1000 nichtinfizierte Zellen. Die Reihe 12 (ohne Substanz) dient als Viruskontrolle. Die End-Testkonzentrationen liegen bei 250 - 0,0005 µM. Die Platten werden 6 Tage bei 37°C / 5% $CO_2$ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque multiplier der Firma Technomara) visuell ausgewertet.
Die folgenden Daten können von den Testplatten ermittelt werden:

$CC_{50}$ (NHDF) = Substanzkonzentration in µM, bei der im Vergleich zur unbehandelten Zellkontrolle keine sichtbaren cytostatischen Effekte auf die Zellen erkennbar sind;

$EC_{50}$ (HCMV) = Substanzkonzentration in µM, die den CPE (cytopathischen Effekt) um 50 % im Vergleich zur unbehandelten Viruskontrolle hemmt;

$$\text{SI (Selektivitätsindex)} = CC_{50}\ \text{(NHDF)} / EC_{50}\ \text{(HCMV)}.$$

Repräsentative in-vitro-Wirkdaten für die erfindungsgemäßen Verbindungen sind in Tabelle A wiedergegeben:

**Tabelle A**

| Beispiel Nr. | NHDF $CC_{50}$ [µM] | HCMV $EC_{50}$ [µM] | SI HCMV |
|---|---|---|---|
| **1** | 15 | 0.1 | 150 |
| **2** | 12 | 0.07 | 171 |
| **3** | 15 | 0.13 | 115 |
| **4** | 8.6 | 0.06 | 143 |
| **5** | 12 | 0.74 | 16 |
| **6** | 12 | 0.35 | 34 |
| **7** | 31 | 1.8 | 17 |
| **32** | 15 | 0.01 | 1500 |
| **36** | 15 | 0.01 | 1500 |

Die Eignung der erfindungsgemäßen Verbindungen zur Behandlung von HCMV-Infektionen kann im folgenden

Tiermodell gezeigt werden:

**HCMV Xenograft-Gelfoam®-Modell**

Tiere:

3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson) bezogen. Die Tiere werden unter sterilen Bedingungen (einschließlich Streu und Futter) in Isolatoren gehalten.

Virusanzucht:

Humanes Cytomegalovirus (HCMV), Stamm Davis, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0,01 werden die virusinfizierten Zellen 5-7 Tage später geerntet und in Gegenwart von Minimal Essential Medium (MEM), 10 % foetalem Kälberserum (FKS) mit 10 % DMSO bei -40˚C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot oder Fixierung und Färbung mit einem Formalin-Giemsa Gemisch (wie unter B. beschrieben).

Vorbereitung der Schwämme, Transplantation, Behandlung und Auswertung:

1x1x1 cm große Kollagenschwämme (Gelfoam®; Fa. Peasel & Lorey, Best.-Nr. 407534; K.T. Chong et al., Abstracts of 39th Interscience Conference on Antimicrobial Agents and Chemotherapy, 1999, S. 439; P.M. Kraemer et al., Cancer Research 1983, (43): 4822-4827) werden zunächst mit Phosphat-gepufferter Saline (PBS) benetzt, die eingeschlossenen Luftblasen durch Entgasen entfernt und dann in MEM + 10 % FKS aufbewahrt. 1 x $10^6$ virusinfizierte NHDF-Zellen (Infektion mit HCMV-Davis M.O.I = 0.01) werden 3 Stunden nach Infektion abgelöst und in 20 µl MEM, 10 % FKS auf einen feuchten Schwamm getropft. Optional werden nach 12-13 Stunden auf die infizierten Schwämme 5 ng/µl basic Fibroblast Growth Factor (bFGF) in 25 µl PBS / 0,1% BSA / 1 mM DTT aufgebracht und 1 Stunde inkubiert. Zur Transplantation werden die immundefizienten Mäuse mit Avertin oder einem Gemisch aus Azepromazin-Xylazin und Ketamin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation werden die Mäuse über einen Zeitraum von 8 Tagen dreimal täglich (7.00 Uhr und 14.00 Uhr und 19.00 Uhr), zweimal täglich (8.00 Uhr und 17.00 Uhr), oder einmal täglich (14.00 Uhr) peroral mit Substanz behandelt. Die Dosis beträgt 3 oder 10 oder 30 oder 100 mg/kg Körpergewicht, das Applikationsvolumen 10 ml/kg Körpergewicht. Die Formulierung der Substanzen erfolgt in Form einer 0,5 %-igen Tylosesuspension optional mit 2 % DMSO. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U / 1,5 ml) aus dem Schwamm freigesetzt und in Gegenwart von MEM, 10 % foetalem Kälberserum, 10 % DMSO bei -140˚C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot oder nach Fixierung und Färbung mit einem Formalin-Giemsa Gemisch (wie unter B. beschrieben). Ermittelt wird die Anzahl infektiöser Viruspartikel nach Substanzbehandlung im Vergleich zur placebobehandelten Kontrollgruppe.

**C. Ausführungsbeispiele für pharmazeutische Zusammensetzungen**

Die erfindungsgemäßen Verbindungen können folgendermaßen in pharmazeutische Zubereitungen überführt werden:

**Tablette:**

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

Herstellung:

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Suspension:**

Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 ml orale Suspension.

Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

## Patentansprüche

**1.** Verbindung der Formel

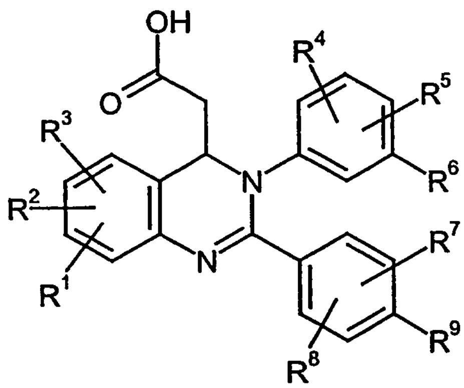

(I),

in welcher

$R^1$, $R^2$ und $R^3$ unabhängig voneinander für Wasserstoff, Alkyl, Alkoxy, Carboxyl, Alkylcarbonyl, Alkoxycarbonyl, Aminocarbonyl, Trifluor- methyl, Halogen, Cyano, Hydroxy oder Nitro stehen,
$R^4$ und $R^5$ unabhängig voneinander für Wasserstoff, Alkyl, Alkoxy, Cyano, Halogen, Nitro, Trifluormethyl oder Trifluormethoxy stehen,
$R^6$ für Alkyl, Cyano, Halogen, Nitro oder Trifluormethyl steht,
$R^7$ und $R^8$ unabhängig voneinander für Wasserstoff, Halogen, Alkyl oder Alkoxy stehen und
$R^9$ für Aryl oder 1,3-Benzodioxol-5-yl steht, worin Aryl und 1,3- Benzodioxol-5-yl substituiert sein können mit 1 bis 3 Substituenten, wobei die Substituenten unabhängig voneinander ausgewählt werden aus der Gruppe, bestehend aus Alkoxy, Alkylthio, Carboxyl, Alkyl- carbonyl, Alkoxycarbonyl, Aminocarbonyl, Trifluormethyl, Halogen, Carbamoyl, Cyano, Hydroxy, Amino, Alkylamino, Nitro und gegebenenfalls Hydroxy-substituiertes Alkyl,

oder eines ihrer Salze, ihrer Solvate oder der Solvate ihrer Salze.

**2.** Verbindung nach Anspruch 1, in welcher

$R^1$, $R^2$ und $R^3$ unabhängig voneinander für Wasserstoff, Fluor, Chlor, Cyano, Hydroxy, Aminocarbonyl oder Nitro stehen,
$R^4$ und $R^5$ unabhängig voneinander für Wasserstoff, Fluor, Alkyl oder Alkoxy stehen,
$R^6$ für Trifluormethyl, iso-Propyl oder tert.-Butyl steht,
$R^7$ und $R^8$ unabhängig voneinander für Wasserstoff, Halogen, $C_1$-$C_3$-Alkyl oder $C_1$-$C_3$-Alkoxy stehen und
$R^9$ für Phenyl oder 1,3-Benzodioxol-5-yl steht, worin Phenyl substituiert sein kann mit 1 bis 3 Substituenten, wobei die Substituenten un- abhängig voneinander ausgewählt werden aus der Gruppe, bestehend aus $C_1$-$C_6$-Alkyl, $C_1$-$C_6$-Alkoxy, Carboxyl, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino und Nitro,

oder eines ihrer Salze, ihrer Solvate oder der Solvate ihrer Salze.

3. Verbindung nach Anspruch 1 oder 2, in welcher

$R^1$ und $R^2$ für Wasserstoff stehen,
$R^3$ für Fluor steht,
$R^4$ und $R^5$ unabhängig voneinander für Wasserstoff, Fluor oder Alkoxy stehen,
$R^6$ für Trifluormethyl steht,
$R^7$ und $R^8$ für Wasserstoff stehen und
$R^9$ für Phenyl steht, worin Phenyl substituiert sein kann mit 1 oder 2 Substituenten, wobei die Substituenten unabhängig voneinander aus- gewählt werden aus der Gruppe bestehend aus Methyl, Methoxy, Fluor und Chlor,

oder eines ihrer Salze, ihrer Solvate oder der Solvate ihrer Salze.

4. Verfahren zur Herstellung einer Verbindung der Formel (I) nach Anspruch 1, **dadurch gekennzeichnet, dass** eine Verbindung der Formel

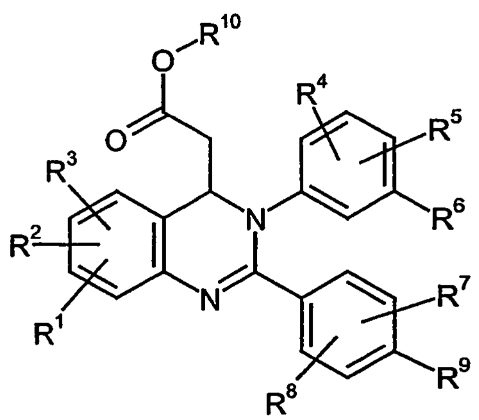

(II),

in welcher

$R^1$, $R^2$, $R^3$, $R^4$, $R^5$, $R^6$, $R^7$, $R^8$ und $R^9$ die in Anspruch 1 angegebene Bedeutung haben, und
$R^{10}$ für Alkyl, bevorzugt Methyl oder Ethyl, steht,

mit einer Base umgesetzt wird.

5. Verbindung nach einem der Ansprüche 1 bis 3 zur Behandlung und/oder Prophylaxe von Krankheiten.

6. Arzneimittel enthaltend eine Verbindung nach einem der Ansprüche 1 bis 3 in Kombination mit einem inerten, nichttoxischen, pharmazeutisch geeigneten Hilfsstoff.

7. Verwendung einer Verbindung nach einem der Ansprüche 1 bis 3 zur Herstellung eines Arzneimittels zur Behandlung und/oder Prophylaxe von Virusinfektionen.

**8.** Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

**9.** Arzneimittel nach Anspruch 6 zur Behandlung und/oder Prophylaxe von Virusinfektionen.

**10.** Verbindung nach einem der Ansprüche 1 bis 3, Arzneimittel nach Anspruch 6 oder ein nach Anspruch 7 oder 8 erhaltenes Arzneimittel zur Verwendung in einem Verfahren zur Bekämpfung von Virusinfektionen in Menschen und Tieren.

## Claims

**1.** Compound of formula

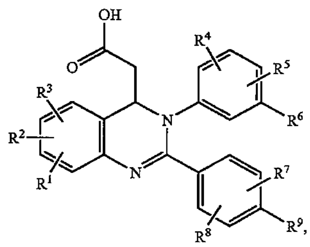

(I),

in which

$R^1$, $R^2$ and $R^3$ independently of one another represent hydrogen, alkyl, alkoxy, carboxy, alkylcarbonyl, alkoxycarbonyl, aminocarbonyl, trifluoro- methyl, halogen, cyano, hydroxy or nitro,
$R^4$ and $R^5$ independently of one another represent hydrogen, alkyl, alkoxy, cyano, halogen, nitro, trifluoromethyl or trifluoromethoxy,
$R^6$ represents alkyl, cyano, halogen, nitro or trifluoromethyl,
$R^7$ and $R^8$ independently of one another represent hydrogen, halogen, alkyl or alkoxy
$R^9$ represents aryl or 1,3-benzodioxol-5-yl, wherein aryl and 1,3- benzodioxol-5-yl may be substituted with 1 to 3 substituents, whereby the substituents are selected independently of one another from the group consisting of alkoxy, alkylthio, carboxy, alkylcarbonyl, alkoxy- carbonyl, aminocarbonyl, trifluoromethyl, halogen, carbamoyl, cyano, hydroxyl, amino, alkylamino, nitro and alkyl which is optionally hy- droxy substituted,

or one of its salts, its solvates or solvates of its salts.

**2.** Compound according to claim 1, in which

$R^1$, $R^2$ and $R^3$ independently of one another represent hydrogen, fluorine, chlorine, cyano, hydroxy, aminocarbonyl or nitro,
$R^4$ and $R^5$ independently of one another represent hydrogen, fluorine, alkyl or alkoxy,
$R^6$ represents trifluoromethyl, iso-propyl or tert-.butyl
$R^7$ and $R^8$ independently of one another represent hydrogen, halogen, $C_1$-$C_3$- alkyl or $C_1$-$C_3$-alkoxy and
$R^9$ represents phenyl or 1,3-benzodioxol-5-yl, wherein phenyl may be substituted with 1 to 3 substituents, whereby the substituents are se- lected independently of one another from the group consisting of $C_1$-$C_6$-alkyl, $C_1$-$C_6$-alkoxy, carboxy, $C_1$-$C_6$-alkylcarbonyl, $C_1$-$C_6$-alkoxy- carbonyl, trifluoromethyl, fluorine, chlorine, bromine, cyano, hydroxy, amino, $C_1$-$C_6$-alkylamino and nitro,

or one of its salts, its solvates or the solvates of its salts.

**3.** Compound according to claim 1 or 2, in which

$R_1$ and $R_2$ represent hydrogen

$R^3$ represents fluorine,
$R^4$ and $R^5$ independently of one another represent hydrogen, fluorine or alkoxy,
$R^6$ represents trifluoromethyl,
$R^7$ and $R^8$ represent hydrogen and
$R^9$ represents phenyl, wherein phenyl may be substituted with 1 or 2 substituents, whereby the substituents are selected independently of one another from the group consisting of methyl, methoxy, fluorine and chlorine,

or one of its salts, its solvates or the solvates of its salts.

**4.** Method for preparing a compound of formula (I) according to claim 1, **characterized in that** a compound of formula

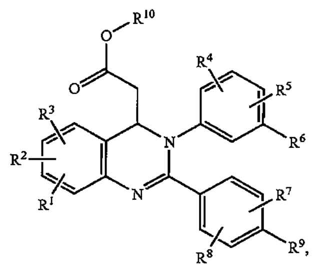

(II),

in which $R^1$, $R^2$, $R^3$, $R^4$, $R^5$, $R^6$, $R^7$, $R^8$ and $R^9$ have the meaning indicated in claim 1, and

$R^{10}$ represents alkyl, preferably methyl or ethyl,

is reacted with a base.

**5.** Compound according to any one of claims 1 to 3 for the treatment and/or prophylaxis of diseases.

**6.** Medicament containing a compound according to any one of claims 1 to 3 in combination with an inert, non-toxic, pharmaceutically acceptable excipient.

**7.** Use of a compound according to any one of claims 1 to 3 for the manufacture of a medicament for the treatment and/or prophylaxis of viral infections.

**8.** Use according to claim 7, **characterized in that** the viral infection is an infection of the human cytomegalovirus (HCMV) or another representative of the group of Herpes viridae.

**9.** Medicament according to claim 6 for the treatment and/or prophylaxis of viral infections.

**10.** Compound according to any one of claims 1 to 3, medicament according to claim 6 or a medicament obtained according to claim 7 or 8 for use in a method for controlling viral infections in humans and animals.

## Revendications

**1.** Composé de formule

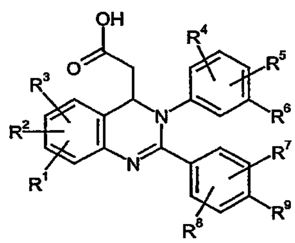

(I),

dans laquelle
$R^1$, $R^2$ et $R^3$ sont indépendamment les uns des autres hydrogène, alkyle, alcoxy, carboxyle, alkylcarbonyle, alcoxycarbonyle, aminocarbonyle, trifluorométhyle, halogène, cyano, hydroxy ou nitro,
$R^4$ et $R^5$ sont indépendamment l'un de l'autre hydrogène, alkyle, alcoxy, cyano, halogène, nitro, trifluorométhyle ou trifluorométhoxy,
$R^6$ est alkyle, cyano, halogène, nitro ou trifluorométhyle,
$R^7$ et $R^8$ sont indépendamment l'un de l'autre hydrogène, halogène, alkyle ou alcoxy, et
$R^9$ est aryle ou 1,3-benzodioxol-5-yle, l'aryle et le 1,3-benzodioxol-5-yle pouvant être substitués par 1 à 3 substituants, lesquels substituants sont choisis indépendamment les uns des autres dans le groupe constitué par alcoxy, alkylthio, carboxyle, alkylcarbonyle, alcoxycarbonyle, aminocarbonyle, trifluorométhyle, halogène, carbamoyle, cyano, hydroxy, amino, alkylamino, nitro, et alkyle facultativement hydroxysubstitué,
ou un de ses sels, de ses solvates ou des solvates de ses sels.

**2.** Composé selon la revendication 1, dans lequel
$R^1$, $R^2$ et $R^3$ sont indépendamment les uns des autres hydrogène, fluor, chlore, cyano, hydroxy, aminocarbonyle ou nitro,
$R^4$ et $R^5$ sont indépendamment l'un de l'autre hydrogène, fluor, alkyle ou alcoxy,
$R^6$ est trifluorométhyle, isopropyle ou tert-butyle,
$R^7$ et $R^8$ sont indépendamment l'un de l'autre hydrogène, halogène, alkyle en $C_1$ à $C_3$ ou alcoxy en $C_1$ à $C_3$, et
$R^9$ est phényle ou 1,3-benzodioxol-5-yle, le phényle pouvant être substitué par 1 à 3 substituants, lesquels substituants sont choisis indépendamment les uns des autres dans le groupe constitué par alkyle en $C_1$ à $C_6$, alcoxy en $C_1$ à $C_6$, carboxyle, alkylcarbonyle en $C_1$ à $C_6$, alcoxycarbonyle en $C_1$ à $C_6$, trifluorométhyle, fluor, chlore, brome, cyano, hydroxy, amino, alkylamino en $C_1$ à $C_6$ et nitro,
ou un de ses sels, de ses solvates ou des solvates de ses sels.

**3.** Composé selon la revendication 1 ou 2, dans laquelle
$R^1$ et $R^2$ sont hydrogène,
$R^3$ est fluor,
$R^4$ et $R^5$ sont indépendamment l'un de l'autre hydrogène, fluor ou alcoxy,
$R^6$ est trifluorométhyle,
$R^7$ et $R^8$ sont hydrogène, et
$R^9$ est phényle, le phényle pouvant être substitué par 1 ou 2 substituants, lesquels substituants sont choisis indépendamment l'un de l'autre dans le groupe constitué par méthyle, méthoxy, fluor et chlore,
ou un de ses sels, de ses solvates ou des solvates de ses sels.

**4.** Procédé de préparation d'un composé de formule (I) selon la revendication 1, **caractérisé en ce que** l'on fait réagir un composé de formule

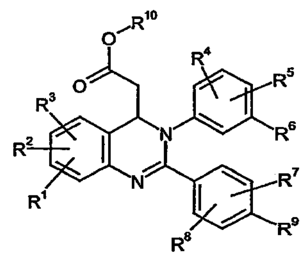

(II),

dans laquelle
$R^1$, $R^2$, $R^3$, $R^4$, $R^5$, $R^6$, $R^7$, $R^8$ et $R^9$ ont les significations indiquées dans la revendication 1, et
$R^{10}$ est alkyle, de préférence méthyle ou éthyle,
avec une base.

**5.** Composé selon l'une des revendications 1 à 3, destiné au traitement et/ou à la prévention de maladies.

**6.** Médicament contenant un composé selon l'une des revendications 1 à 3 en combinaison avec un excipient pharmaceutiquement approprié non toxique et inerte.

**7.** Utilisation d'un composé selon l'une des revendications 1 à 3 pour fabriquer un médicament destiné au traitement et/ou à la prévention d'infections virales.

**8.** Utilisation selon la revendication 7, **caractérisée en** te que l'infection virale est une infection par le cytomégalovirus humain (CMVH) ou par un autre représentant du groupe des herpèsviridés.

**9.** Médicament selon la revendication 6, destiné au traitement et/ou à la prévention d'infections virales.

**10.** Composé selon l'une des revendications 1 à 3, médicament selon la revendication 6, ou médicament obtenu selon la revendication 7 ou 8, destinés à être utilisés dans un procédé de lutte contre des infections virales chez l'homme et les animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Saito T. et al.** *Tetrahedron Lett.,* 1996, vol. 37, 209-212
- **K.T. Chong et al.** *Abstracts of 39th Interscience Conference on Antimicrobial Agents and Chemotherapy,* 1999, 439
- **P.M. Kraemer et al.** *Cancer Research,* 1983, 4822-4827